# EUROPEAN PATENT APPLICATION

(11) **EP 4 825 811 A1**
(43) Date of publication of application: **21.10.2026**
(21) Application number: 26172908.1
(22) Date of filing: 16.04.2026
(51) Int. Cl.: C02F 1/00, C02F 1/50, E04H 4/12

(54) **POOL ANTIBACTERIAL DEVICE AND POOL**

(30) Priority: 18.04.2025 CN 202520752676 U
(71) Applicant: Bestway Inflatables & Material Corp., Shanghai 201812 (CN)
(72) Inventor: ZENG, Xianglin, SHANGHAI, 201812 (CN); SONG, Qingshui, SHANGHAI, 201812 (CN); HU, Wenhua, SHANGHAI, 201812 (CN)
(74) Representative: Inchingalo, Simona

(57) **Abstract**

A pool antibacterial device includes a housing having a first opening and a second opening, and a copper substrate arranged in the housing and including a mesh structure which allows a water flow to pass therethrough. The mesh structure is in fluid communication with the first opening and the second opening.

## Description

### TECHNICAL FIELD

The present disclosure relates to the technical field of pool disinfection and antibacterial treatment, and in particular to a pool antibacterial device and a pool.

### BACKGROUND

The cleanliness and hygiene of water in a pool are directly related to the health and safety of a user. Therefore, in order to ensure the cleanliness of the water in the pool, the pool may be disinfected to aid in inhibiting bacterial growth. Traditional antibacterial approaches for above-ground swimming pools include chlorine tablet dispensing and copper ion electrolysis systems. For example, chlorine tablets may be dispensed in a floating dispenser and may slowly dissolve in water by means of the floating dispenser to release active chlorine to achieve a bactericidal effect. However, this approach requires frequent dispensing of the chlorine tablets, and the chlorine tablets may produce a pungent odor that negatively impacts the user experience. In addition, by products such as chloramine produced by the reaction of chlorine tablets in water can, in some instances, cause harm to the human body.

In contrast, traditional copper ion electrolysis systems utilize electric current to release copper ions from a copper electrode to achieve an antibacterial effect. Although this approach reduces the dispensing frequency and eliminates issues with pungent odors, it is difficult to control and maintain the concentration of copper ion within a standard range, which may = pose a safety hazard.

Therefore, there is a need for a safer, more environmentally friendly and lower-maintenance solution for disinfecting water and preventing bacteria growth in pools.

### SUMMARY

An objective of the present disclosure is to solve the safety hazards and frequent operation problems of existing pool antibacterial approaches. The present disclosure provides a pool antibacterial device and a pool that can reduce safety hazards and require no frequent operation while ensuring the disinfection and antibacterial effect of a pool.

In order to solve the above technical problem, an implementation of the present disclosure discloses a pool antibacterial device, including: a housing, including a first opening and a second opening; at least a copper substrate arranged in the housing and including a mesh structure which allows a water flow to pass therethrough, the mesh structure being in fluid communication with the first opening and the second opening. The copper substrate is placed between the first opening and the second opening.

In some embodiments, the housing includes: a fixing base including the first opening; and a first housing connected to the fixing base to define a first receiving chamber and including the second opening; and the pool antibacterial device further includes: a support frame arranged in the first receiving chamber, connected to the fixing base and in fluid communication with the first opening. The support frame has a grid structure; and the copper substrate is placed inside the first housing, arranged around the support frame, and the mesh structure of the copper substrate is in fluid communication with the grid structure and with the second opening.

In some embodiments, the housing includes: an upper housing including the first opening; and a lower housing connected to the upper housing to form a second receiving chamber and including the second opening; and the copper substrate is arranged in the second receiving chamber, between the fist opening and the second opening, and at least part of the copper substrate is in contact with an inner wall of the second receiving chamber.

In some embodiments, the housing includes: a second housing, including the first opening and the second opening; and a cover sealingly connected to the second housing to define a third receiving chamber; and the pool antibacterial device further includes: a plurality of water distribution plates spaced apart from each other in a vertical direction and arranged in the third receiving chamber, the copper substrate being arranged between adjacent water distribution plates of the plurality of water distribution plates; a communication tube for connecting the plurality of water distribution plates, where a water distribution plate of the plurality of water distribution plates that is close to the cover and the cover jointly define a first water chamber, the first water chamber being in fluid communication with the first opening of the second housing via the communication tube; and a water distribution plate of the plurality of water distribution plates that is close to a bottom wall of the third receiving chamber and the bottom wall of the third receiving chamber jointly define a second water chamber, the second water chamber being in fluid communication with the second opening and in fluid communication with the first water chamber via the mesh structure of the copper substrate.

In some embodiments, the pool antibacterial device further includes: a fibrous filter material arranged between certain adjacent water distribution plates of the plurality of water distribution plates and around the communication tube, where the first water chamber is in fluid communication with the second water chamber via the mesh structure of the copper substrate and the fibrous filter material.

In some embodiments, a support portion protrudes from the bottom wall of the third receiving chamber and supports the water distribution plate of the plurality of water distribution plates that is adjacent to the bottom wall.

In some embodiments, each of the plurality of water distribution plates includes a mounting portion, the mounting portion including one of a groove and a protrusion; and an outer wall of the communication tube includes the other of the groove and the protrusion, and the communication tube is connected to each of the plurality of water distribution plates through the cooperation of the groove and the protrusion.

In some embodiments, each of the plurality of water distribution plates includes a fixing recess in which the copper substrate is arranged; and a bottom wall of the fixing recess includes a plurality of through holes, the copper substrates adjacent to each other in the vertical direction are in fluid communication via the plurality of through holes, and/or the fibrous filter material adjacent to the copper substrate in the vertical direction is in fluid communication with the copper substrate via the plurality of through holes.

The present disclosure also discloses a pool based on any of the above implementations, the pool including: a pool body, including a side wall and a bottom wall, the side wall and the bottom wall jointly defining a water cavity, and the side wall being provided with a pool water inlet and a pool water outlet; a water pump arranged outside the water cavity and including a pump water inlet and a pump water outlet, the pump water inlet being in fluid communication with the pool water outlet, and the pump water outlet being in fluid communication with the pool water inlet; and a pool antibacterial device as described above, the pool antibacterial device being mounted to the pool water inlet and/or the pool water outlet, the pool water inlet and/or the pool water outlet being in communication with the first opening, and the second opening being located in the water cavity.

The present disclosure also discloses a pool based on any of the above implementations, the pool including: a pool body, including a side wall and a bottom wall, the side wall and the bottom wall jointly defining a water cavity, and the side wall being provided with a pool water inlet and a pool water outlet; a water pump arranged outside the water cavity and including a pump water inlet and a pump water outlet; an external piping including a first water pipe, a second water pipe, and a third water pipe; and a pool antibacterial device as described above, the pool antibacterial device being mounted on the external piping,
where the pump water inlet is in fluid communication with the pool water outlet via the first water pipe, the pump water outlet is in fluid communication with the first opening of the pool antibacterial device via the second water pipe, and the second opening of the pool antibacterial device is in fluid communication with the pool water inlet via the third water pipe.

The present disclosure also discloses a pool based on any of the above implementations, the pool including: a pool body, including a side wall and a bottom wall, the side wall and the bottom wall jointly defining a water cavity, and the side wall being provided with a pool water inlet and a pool water outlet; a water pump arranged outside the water cavity and including a pump water inlet and a pump water outlet; an external piping including a first water pipe, a second water pipe, and a third water pipe; and a pool antibacterial device as described above, the pool antibacterial device being mounted between the pump water outlet and the pool water inlet, where the pump water inlet is in fluid communication with the pool water outlet via the first water pipe, the pump water outlet is in fluid communication with the first opening of the pool antibacterial device via the second water pipe, and the second opening of the pool antibacterial device is in fluid communication with the pool water inlet via the third water pipe.

In the pool antibacterial device of the present disclosure, the copper substrate is employed, which is mounted in the housing, and the two openings (i.e., the first opening and the second opening) are in fluid communication with each other through the mesh structure of the copper substrate, so that a water flow is in full contact with the copper substrate as the water flow passes through the device. The pool antibacterial device does not require an external power source, the copper substrate generates a potential difference on the surface of the copper substrate under the action of the water flow, and in turn continuously releases copper ions slowly. The copper ions enter the pool with the water flow and gradually accumulate in the pool. When the concentration of the copper ions reaches a set value (e.g., greater than 0.3 PPM), the copper ions can bind to cell membranes of microorganisms such as algae in the pool, to disrupt their structure and function, thereby effectively inhibiting the growth of the microorganisms and achieving the effect of continuous antibacterial, algae inhibition and water purification.

### BRIEF DESCRIPTION OF THE DRAWINGS

Other features and advantages of the present disclosure will be understood from the following embodiments described in detail herein and with reference to the accompanying drawings, in which like reference numerals represent the same or similar components.
FIG. 1 shows a schematic view of a pool antibacterial device mounted to a pool water outlet, according to one or more embodiments of the present disclosure;
FIG. 2 shows a schematic view of the pool antibacterial device of FIG. 1 mounted to a pool water inlet, according to one or more embodiments of the present disclosure;
FIG. 3 shows a perspective view of the pool antibacterial device of FIG. 1, according to one or more embodiments of the present disclosure;
FIG. 4 shows a cross-sectional view of the pool antibacterial device shown in FIG. 3, according to one or more embodiments of the present disclosure;
FIG. 5 shows an exploded view of the pool antibacterial device shown in FIG. 3, according to one or more embodiments of the present disclosure;
FIG. 6 shows a perspective view of a first frame portion and a second frame portion of the pool antibacterial device shown in FIG. 3, according to one or more embodiments of the present disclosure;
FIG. 7 shows a cross-sectional view of the pool antibacterial device shown in FIG. 3, according to one or more embodiments of the present disclosure;
FIG. 8 shows a schematic view of another embodiment of a pool antibacterial device mounted to a pool water outlet, according to one or more embodiments of the present disclosure;
FIG. 9 shows a schematic view of the pool antibacterial device of FIG. 8 mounted to a pool water inlet, according to one or more embodiments of the present disclosure;
FIG. 10 shows a schematic view of the pool antibacterial device of FIG. 8 mounted to an external piping of the pool, according to one or more embodiments of the present disclosure;
FIG. 11 shows a cross-sectional view of the pool antibacterial device of FIG. 8, according to one or more embodiments of the present disclosure;
FIG. 12 shows an exploded view of the pool antibacterial device of FIG. 8, according to one or more embodiments of the present disclosure;
FIG. 13 shows a schematic view of another embodiment of a pool antibacterial device mounted to an external piping of the pool, according to one or more embodiments of the present disclosure;
FIG. 14 shows a perspective view of the pool antibacterial device of FIG. 13, according to one or more embodiments of the present disclosure;
FIG. 15 shows an exploded view of the pool antibacterial device shown in FIG. 14, according to one or more embodiments of the present disclosure;
FIG. 16 shows a cross-sectional view of the pool antibacterial device shown in FIG. 14, according to one or more embodiments of the present disclosure;
FIG. 17 shows a partially enlarged view of portion A of the pool antibacterial device shown in FIG. 16, according to one or more embodiments of the present disclosure;
FIG. 18 shows a perspective view of a third housing of the pool antibacterial device shown in FIG. 14, according to one or more embodiments of the present disclosure;
FIG. 19 shows a perspective view of a communication tube and a water distribution plate of the pool antibacterial device shown in FIG. 14, according to one or more embodiments of the present disclosure;
FIG. 20 shows a perspective view of a plurality of water distribution plates of the pool antibacterial device shown in FIG. 14, according to one or more embodiments of the present disclosure;
FIG. 21 shows an exploded view of another embodiment of a pool antibacterial device, according to one or more embodiments of the present disclosure; and
FIG. 22 shows a cross-sectional view of the pool antibacterial device shown in FIG. 21, according to one or more embodiments of the present disclosure.

### DETAILED DESCRIPTION

Embodiments of the present disclosure are illustrated below, and those skilled in the art may readily understand advantages and effects of the present disclosure from the content disclosed in the description. Although the description of the present disclosure will be introduced in conjunction with preferred embodiments, it does not mean that features of the present disclosure are limited to the implementations described herein. On the contrary, an objective of introducing the present disclosure in conjunction with the embodiments described herein is to encompass other options or modifications that may be extended on the basis of the claims of the present disclosure. The following description contains numerous specific details in order to provide deep understanding of the present disclosure. The present disclosure may also be implemented without these details. In addition, in order to avoid confusion of the present disclosure, some specific details will be omitted in the description. It should be noted that the embodiments and the features thereof in the present disclosure can be combined with each other without conflicts.

In the description of the present disclosure, it should be understood that orientation or position relationships indicated by terms such as "center", "longitudinal", "transverse", "length", "width", "thickness", "up", "down", "front", "rear", "left", "right", "vertical", "horizontal", "top", "bottom", "inside", "outside", "clockwise", and "anticlockwise" are based on orientation or position relationships shown in the accompanying drawings and are merely for ease of description of the present disclosure and simplification of the description, rather than indicating or implying that the apparatuses or elements referred to must have a specific orientation or be constructed and operated in a specific orientation, and therefore cannot be construed as limiting the present disclosure.

In addition, terms "first", "second", etc. are merely for the purpose of description, and should not be construed as indicating or implying the relative importance or implicitly indicating the number of technical features indicated. Thus, the features defined with "first" and "second" may explicitly or implicitly include one or more features. In the description of the present disclosure, the meaning of "a plurality of" is two or more, unless specifically defined otherwise.

In the present disclosure, it should also be noted that the terms "arrange", "connected", and "connection" should be understood in a broad sense, unless otherwise explicitly specified and limited. For example, the connection may be a secured connection, a detachable connection, or an integral connection; or may be a mechanical connection or an electrical connection; or may be a direct connection, an indirect connection by means of an intermediate medium, or internal communication between two elements. For those of ordinary skill in the art, the specific meaning of the terms mentioned above in the embodiments should be understood in specific cases.

In the present disclosure, unless otherwise explicitly specified and defined, the first feature being "above" or "below" the second feature may include the first and second features being in direct contact, or may include the first and second features being not in direct contact but coming into contact through another feature between them. In addition, the first feature being "above", "over", and "on" the second feature includes the first feature being directly above and obliquely above the second feature, or simply means that the level of the first feature is higher than that of the second feature. The first feature being "below", "beneath", and "on underside of" the second feature includes the first feature being directly below and obliquely below the second feature, or simply means that the level of the first feature is less than that of the second feature.

In the description of the present disclosure, the meaning of "a plurality of" is two or more, unless specifically defined otherwise.

To make the objectives, technical solutions, and advantages of the present disclosure clearer, the technical solutions of the present disclosure will be clearly and completely described with reference to specific embodiments of the present disclosure and corresponding figures.

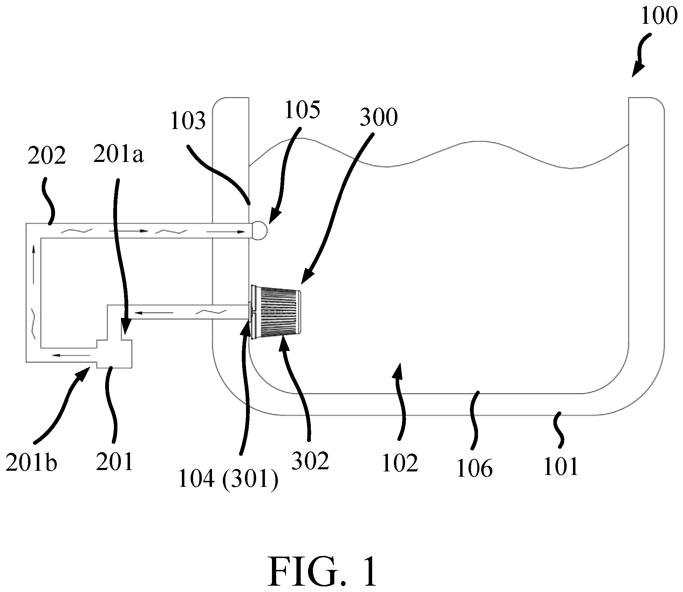

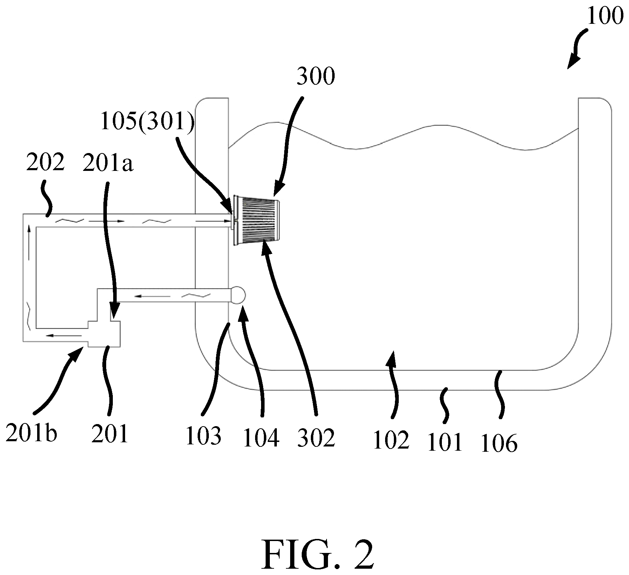

Referring now to FIGS. 1 and 2, the embodiments of the present disclosure depict a pool 100. The pool 100 may include a pool body 101. The pool body 101 may include a water cavity 102. The water cavity 102 may be defined jointly by a side wall 103 and a bottom wall 106 of the pool body 101. That is, the side wall 103 and the bottom wall 106 may form an enclosure that defines the water cavity 102. The water cavity 102 may be configured to store water to meet a user's needs for entertainment, fitness, exercise, and/or other similar leisure activities. In these embodiments, the side wall 103 may be provided with a pool water outlet 104 and a pool water inlet 105.

It should be appreciated that, in the embodiments described herein, the pool 100 may be an above-ground pool or an underground pool (e.g., in-ground pool). Furthermore, in embodiments in which the pool 100 is an above-ground pool, the pool 100 may include an inflatable pool, a massage pool, a frame pool, or any other similar pool without departing from the scope of the present disclosure

As provided herein, it should be further understood that the term "pool water outlet 104" may refer to a water port through which water flows from the water cavity 102 to an external environment, and the term "pool water inlet 105" may refer to a water port through which water flows from the external environment and into the water cavity 102.

Referring again to FIG. 1, the pool 100 may further include a water pump 201. The water pump 201 may be arranged outside the water cavity 102 of the pool body 101, and two ends of the water pump 201 are in fluid communication with the pool water outlet 104 and the pool water inlet 105, respectively, via an external piping 202. Therefore, the water pump 201 is preferably mounted to the external piping 202. For example, in these embodiments, the water pump 201 may include a pump water inlet 201a and a pump water outlet 201b. The pump water inlet 201a may be in fluid communication with the pool water outlet 104, and the pump water outlet 201b may be in fluid communication with the pool water inlet 105. That is, the water pump 201 and the water cavity 102 may jointly form a water circulation flow path, and the water pump 201 may draw water from the water cavity 102 through the pool water outlet 104, and the water may be re-injected into the water cavity 102 through the pool water inlet 105, thereby realizing the circulation flow of the water within the water cavity 102.

When the pool 100 is in use (e.g., for recreation, leisure, fitness or exercise purposes), microorganisms such as bacteria and viruses that may be carried by the human body may enter the pool 100. Furthermore, dust and other impurities from the external environment may also be introduced into the pool 100, particularly via the flow of air or when the pool 100 is replenished of water. It should be appreciated that, in these embodiments, if algae and microorganisms proliferate extensively in the water cavity 102, the water will become turbid and produce an odor, and the water quality will deteriorate. Accordingly, microorganisms may not only impact on the aesthetics and user experience of the pool 100, but may also introduce potential risks to the health of the user. Therefore, in order to ensure the cleanliness of the water of the pool 100 and to protect the health of the user, sterilization and antibacterial treatment of the water may be provided.

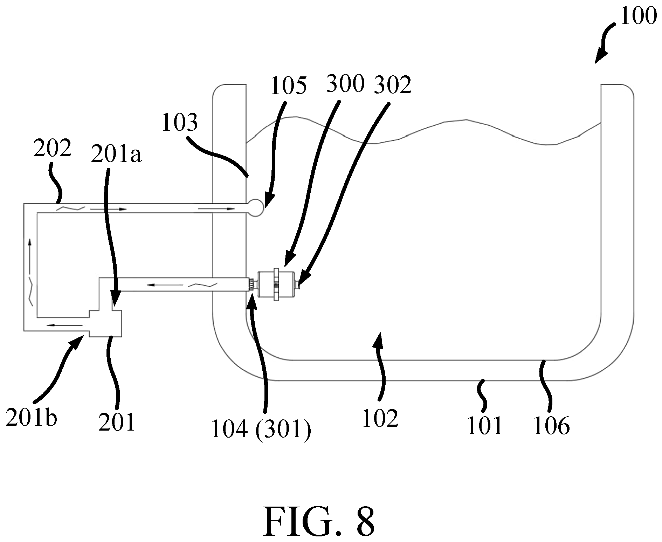

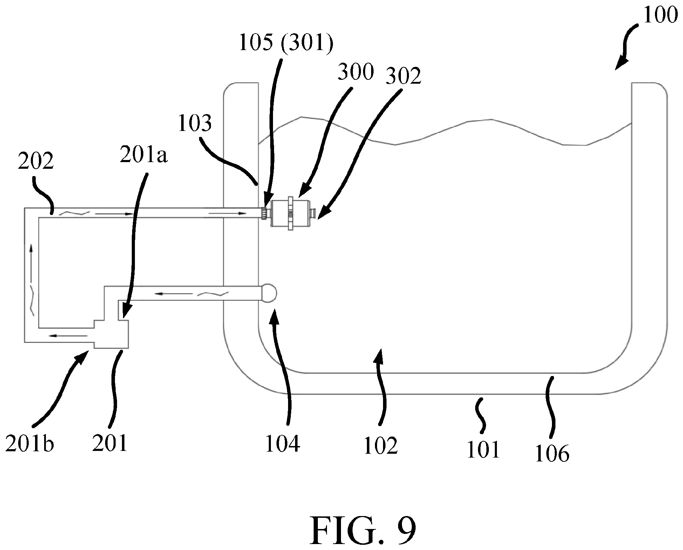

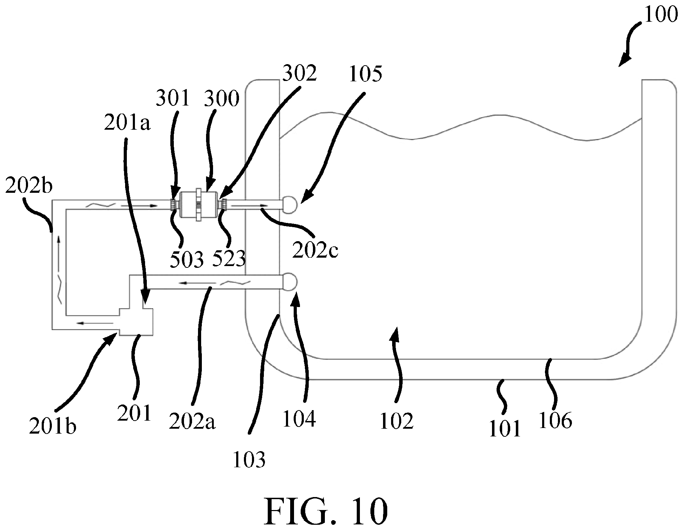

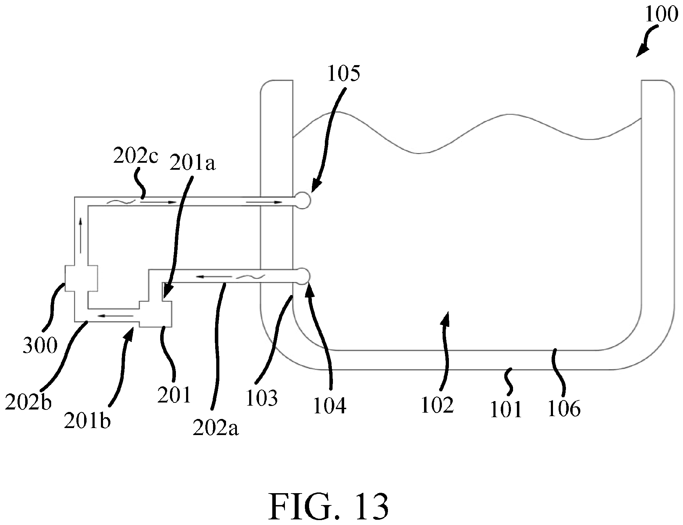

To remedy issues associated with microorganisms and other bacteria, the pool 100 depicted in FIGS. 1 and 2 may further include a pool antibacterial device 300, which may be mounted to the pool water outlet 104 (shown in FIGS. 1 and 8), or to the pool water inlet 105 (shown in FIGS. 2 and 9), or to each of the pool water outlet 104 and the pool water inlet 105 (not shown). In these embodiments, the pool antibacterial device 300 may be connected to a water circulation path formed between the water pump 201 and the water cavity 102, and may effectively inhibit the growth of bacteria in the pool 100 through antibacterial treatment of the water during the water circulation process, thereby ensuring that the water quality of the pool 100 remains clean while maintaining the sanitary safety of users of the pool. As noted hereinabove, the pool antibacterial device 300 may be mounted to the pool water outlet 104 and to the pool water inlet 105, with each embodiment being described in additional detail herein. Another structural way to connect the pool antibacterial device 300 to a pool, described in detail below, requires that the pool antibacterial device 300 to be mounted to the external piping 202 (shown in FIGS. 10 and 13) between the pool water outlet 104 and the pool water inlet 105.

In the following paragraphs the pool antibacterial device 300 will be described in detail, with reference to each embodiment disclosed in the present invention. In general, the pool antibacterial device 300 comprises a housing 430, 500, 810 with a first opening 301 and a second opening 302. The pool antibacterial device 300 is in fluid communication with the water cavity 102 through such first opening 301 and second opening 302. The pool antibacterial device 300 comprises at least a copper substrate 304 placed within the housing 430, 500, 810 and located between the first opening 301 and the second opening 302. The copper substrate 304 comprises a mesh structure 306 that is designed to allow water to pass through it for antibacterial treatment. The first opening 301 and the second opening 302 are in fluid communication with each other via the mesh structure 306 of the copper substrate 304. This allows the water to be treated to exit the pool water cavity 102 and enter the pool antibacterial device 300 via the first opening 301 (or the second opening 302). It then passes across the mesh structure 306 of the copper substrate 304, where it is treated and purified, and exits through the second opening 302 (or the first opening 301 if the water first enters through the second opening 302) to enter again into the water cavity 102 of the pool.

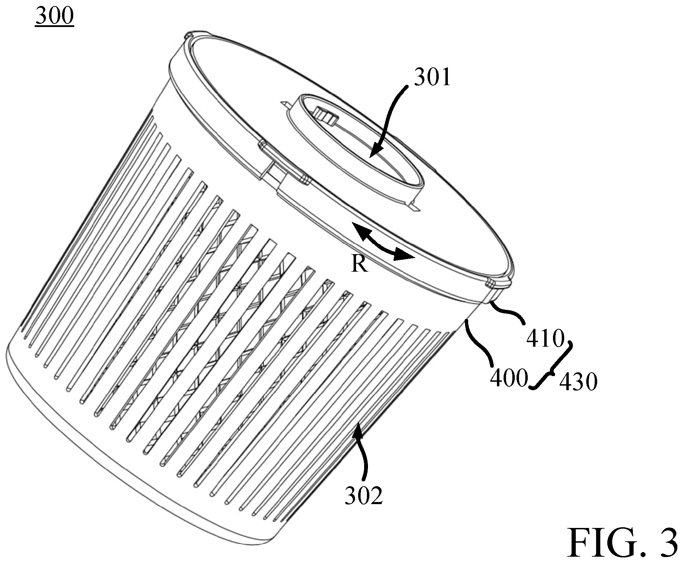

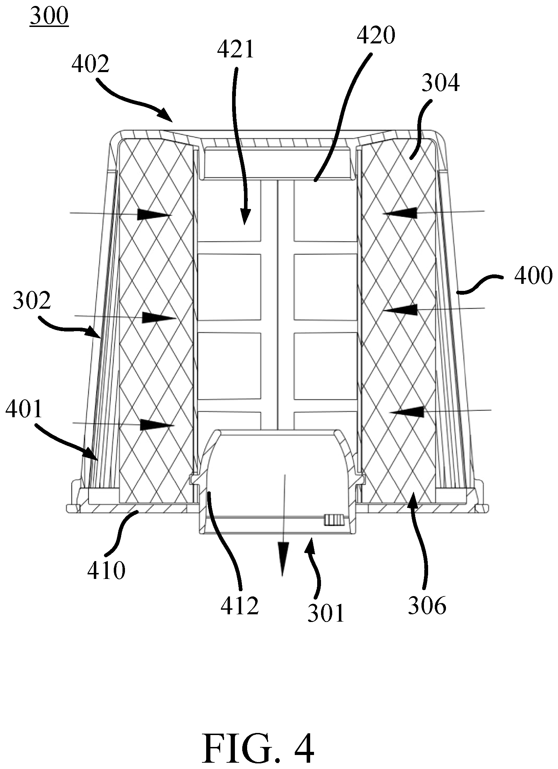

Referring now to FIGS. 3 and 4, the pool antibacterial device 300 may further include a support frame 420 and a housing 430. The housing 430 may include a first housing 400 and a fixing base 410. The first housing 400 may include a second opening 302 disposed on a side wall of the first housing 400. The fixing base 410 may include a first opening 301, and the fixing base 410 may be connected to the first housing 400 to define a first receiving chamber 401. The first opening 301 may be configured for communication with the pool water outlet 104 or the pool water inlet 105, and the second opening 302 may be located in the water cavity 102 for circulation of the water.

In some embodiments, the first housing 400 may be of a truncated cone-shaped structure, and the second opening 302 may be of an elongated rectangular structure. A plurality of second openings 302 may be spaced apart from each other in a circumferential direction R of the first housing 400 and arranged on the side wall of the first housing 400, to facilitate even distribution and efficient treatment of the water flow within the first housing 400. Although FIGS. 3 and 4 depict the first housing 400 as including the truncated cone-shaped structure and the second opening 302 as including the elongated rectangular structure, it should be appreciated that the various components of the housing may have any shape without departing from the scope of the present disclosure.

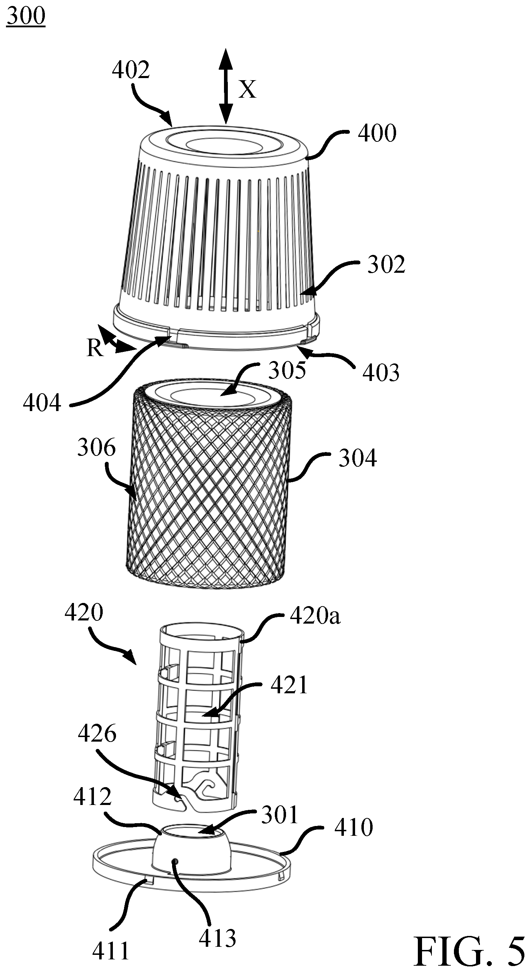

Turning now to FIG. 5, the first housing 400 may include a closed end 402 and an open end 403, with the closed end 402 and the open end 403 being spaced apart from one another in an axial direction X. In these embodiments, the open end 403 may be connected to the fixing base 410, and the fixing base 410 may have a circular structure shaped and dimensioned to adapt to the open end 403 of the first housing 400. As further illustrated in FIG. 5, the open end 403 of the first housing 400 may be provided with a first engagement recess 404, and the fixing base 410 may be provided with a first projection 411 which may be engaged with the first engagement recess 404, such that a reliable connection between the fixing base 410 and the first housing 400 may be achieved.

It should be further appreciated that, in some embodiments, the fixing base 410 and the first housing 400 may be connected by other means, such as ultrasonic welding, threaded connection, snap-fit, connection, or any other similar connection means without departing from the scope of the present disclosure. Furthermore, the connection means implemented in the fixing base 410 and the first housing 400 may be selected according to the disclosure and are not limited to the particular embodiments described herein.

Referring now to FIGS. 4 and 5, a support frame 420 and a copper substrate 304 may be disposed in the first housing 400, in particular in the first receiving chamber 401 described above. In detail, the support frame 420 is connected to the fixing base 410 and is in fluid communication with the first opening 301, The support frame 420 may have a grid structure 420a, and the copper substrate 304 may be arranged around the outside of the support frame 420. As shown in FIGS. 4 and 5, the grid structure 420a of the support frame 420 is preferably coaxial with the first opening 301. The second opening 302 is located in the lateral wall of the first housing 400, so the mesh structure 306 of the copper substrate 304 remains positioned between the first and second openings. In the embodiment depicted in FIGS. 4 and 5, the grid structure 420a of the support frame 420 may have a hollow cylindrical shape, and the copper substrate 304 also may have a hollow cylindrical shape and may be provided with a central hole 305. The copper substrate 304 may be sleeved outside the support frame 420, such that the copper substrate 304 and the support frame 420 fit against each other, thereby enabling the support frame 420 to support and position the copper substrate 304. It should be further appreciated that, in some embodiments, the structural form of the copper substrate 304 and the support frame 420 may not be limited to the hollow cylindrical shape and may include any shape without departing from the scope of the present disclosure. For example, the copper substrate 304 and the support frame 420 may have other geometries, such as a truncated cone, a cuboid, or a square, so long as the copper substrate 304 and the support frame 420 can securely cooperate with each other to enable the support frame 420 to support and position the copper substrate 304.

Referring still to FIGS. 4 and 5, a hollow cylindrical portion of the support frame 420 may form a water flow passage 421, and the grid structure 420a of the support frame 420 may fit against a hole wall of the central hole 305 of the copper substrate 304, so that the water in the water flow passage 421 may be directly in contact with the copper substrate 304.

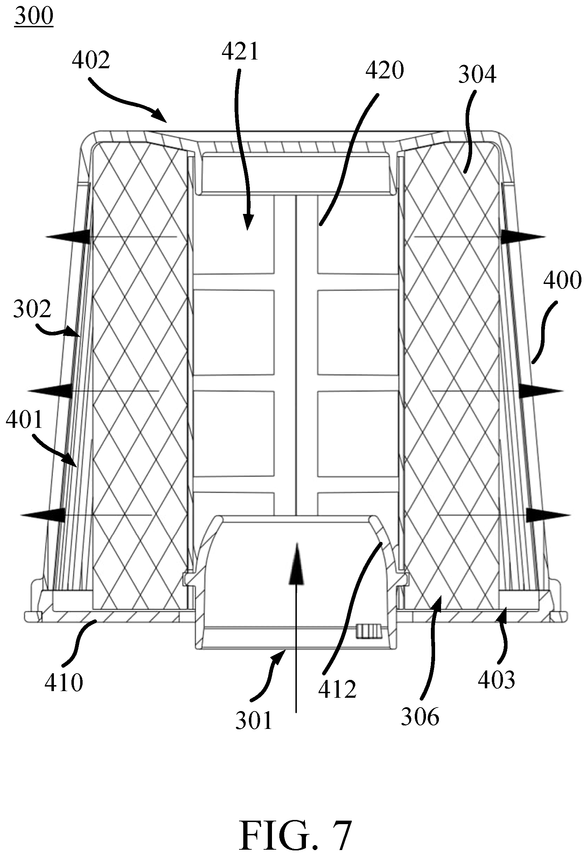

As already stated, the copper substrate 304 may further include a mesh structure 306 that may allow water to flow therethrough. The mesh structure 306 is in fluid communication with the grid structure 420a so that water in the water flow passage 421 can flow into or out of the water cavity 102 of the pool 100 through the grid structure 420a and the mesh structure 306 of the copper substrate 304. It should be further appreciated that, as shown in FIG. 4 (e.g., in which the pool antibacterial device 300 is mounted to the pool water outlet 104), the water in the water cavity 102 may flow into the pool antibacterial device 300 through the second opening 302 of the first housing 400, and sequentially pass through the mesh structure 306 of the copper substrate 304 and the grid structure 420a, so as to flow into the water pump 201 through the first opening 301 of the fixing base 410. In these embodiments, the flow configuration of water through water cavity 102, pool antibacterial device 300, and water pump 201 may achieve the circulation and antibacterial effect of the water. Furthermore, in another embodiment, as shown in FIG. 7, in which the pool antibacterial device 300 may be mounted to the pool water inlet 105, after flowing from the first opening 301 into the pool antibacterial device 300, the water flow may sequentially pass through the grid structure 420a and the mesh structure 306 of the copper substrate 304, and flow into the water cavity 102 through the second opening 302 in the first housing 400, to achieve the circulation and antibacterial effect of the water.

It should be further understood that, in the embodiments described herein, the mesh structure 306 of the copper substrate 304 may be formed by a plurality of copper wires interlaced, connected, and woven to form a structure having a plurality of interconnected pores or cavities, to ensure that the water flow smoothly flows through the copper substrate 304.

The first opening 301 in the fixing base 410 described above may also be in fluid communication with the water flow passage 421, and the second opening 302 may be in fluid communication with the water flow passage 421 through the mesh structure 306 of the copper substrate 304. That is, regardless of a direction of the water flow (e.g., from the first opening 301 to the second opening 302 or from the second opening 302 to the first opening 301), the water flow may pass through the copper substrate 304 to facilitate the functioning of the copper substrate 304 in the water flow path, so that the copper substrate may generate a potential difference on the surface of the copper substrate 304 and continuously releases copper ions.

It should be further appreciated that, during the process of the water flow passing through the copper substrate 304, the copper substrate 304 may generate a potential difference on the surface of the copper substrate, the copper substrate 304 may gradually release copper ions, and the copper ions may flow into the water cavity 102 of the pool 100 with the water flow. When the concentration of the copper ions in the water cavity 102 reaches a set value (e.g., greater than 0.3 PPM), the copper ions may bind to the cell membranes of the algae or other microorganisms within the water cavity 102, disrupting their structure and function, thereby causing the death of the microorganisms, effectively inhibiting the growth of colonies in the pool, and achieving the effects of antibacterial and water cleaning.

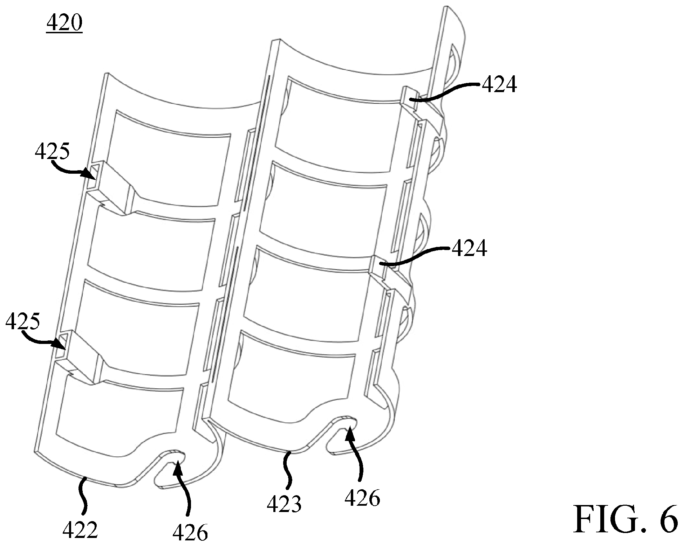

Referring now to FIGS. 5 and 6, in some embodiments, the support frame 420 may include a first frame portion 422 and a second frame portion 423, with each of the first frame portion 422 and the second frame portion 423 including a semi-cylindrical shape. One end of the first frame portion 422 may be hinged to one end of the second frame portion 423, the other end of the first frame portion 422 may be provided with a second engagement recess 425 and, correspondingly, the other end of the second frame portion 423 may be provided with a second projection 424. The second projection 424 may be engaged with the second engagement recess 425, to enable a removable connection between the first frame portion 422 and the second frame portion 423. In this embodiment, two second projections 424 and two second engagement recesses 425 are provided and are spaced apart from each other in the axial direction X of the support frame 420, so as to ensure the reliability of the connection between the first frame portion 422 and the second frame portion 423.

In the embodiments described herein, the number of second projections 424 and the number of second engagement recesses 425 may be set to one, three, four or any other number of projections and/or engagement recesses without departing from the scope of the present disclosure. Furthermore, in some embodiments, the first frame portion 422 and the second frame portion 423 may be connected by other connection means, such as snap connection, screw connection, or any other similar connection configured to achieve the same removable connection function.

As shown in FIG. 5, the fixing base 410 may include a boss 412 disposed on the surface of the fixing base 410, the boss 412 being provided with a third projection 413 for engagement, and a lower end of the support frame 420 may be provided with a slot 426 for cooperating with the third projection 413. When assembled, the lower end of the support frame 420 may be sleeved on an outer side of the boss 412, and the third projection 413 may slide into the slot 426, thereby achieving the limiting and fixing of the support frame 420 with respect to the fixing base 410. Such an engagement structure may enable strong detachability and easy assembly.

Furthermore, as a result of the removable connection structure formed between the first frame portion 422 and the second frame portion 423, before the frame portions are fully assembled, the slot 426 of the support frame 420 may be aligned with the third projection 413 on the fixing base 410 for slidable mounting, and after the completion of the initial connection with the fixing base 410, the stable assembly of the support frame 420 as a whole may be achieved by the engagement of the second projection 424 with the second engagement recess 425.

It should be further understood that the support frame 420 and the fixing base 410 may be connected by other means, such as snap-fit connection, bolt fastening, or threaded connections, to meet different mounting and dismounting requirements, without departing from the scope of the present disclosure.

Referring now to FIGS. 1, 4 and 5, when the pool antibacterial device 300 is mounted to the pool water outlet 104, a mounting base (not shown) may be disposed at the pool water outlet 104, and the fixing base 410 of the pool antibacterial device 300 may be connected to the mounting base so that the pool antibacterial device 300 may be fixed at the pool water outlet 104. In particular, the first opening 301 of the pool antibacterial device 300 may be butt-jointed to and in fluid communication with the pool water outlet 104, and the second opening 302 of the pool antibacterial device 300 may be submerged within the water cavity 102 of the pool 100. During use, when driven by the water pump 201, the water in the water cavity 102 may enter the pool antibacterial device 300 through the second opening 302 and may sequentially flow through the copper substrate 304 and the water flow passage 421 of the support frame 420. During this process, the copper substrate 304 may generate a potential difference on the surface of the copper substrate due to contact with the water flow, and in turn, may gradually release copper ions. The copper ions may enter the water flow passage 421 with the water flow and finally flow out of the pool antibacterial device 300 through the first opening 301. The water flow may be conveyed to the pool water inlet 105 through the external piping 202 and may flow return to the water cavity 102 of the pool 100, to achieve the circulation treatment of the water. When the concentration of the copper ions in the water cavity 102 reaches a set value (e.g., greater than 0.3 PPM), the copper ions bind to the cell membranes of the algae or other microorganisms in the water, disrupting their structure and function, thereby causing the death of the microorganisms, effectively inhibiting the growth of microbial colonies in the pool, and achieving the antibacterial and cleaning effect of the pool.

Turning now to FIGS. 2, 5 and 7, in another embodiment, the pool antibacterial device 300 may be mounted to the pool water inlet 105, a mounting base (not shown) may be also disposed at the pool water inlet 105, and the fixing base 410 of the pool antibacterial device 300 may be connected to the mounting base so that the pool antibacterial device 300 may be fixed at the pool water inlet 105. Specifically, the first opening 301 of the pool antibacterial device 300 may be butt-jointed to and in fluid communication with the pool water inlet 105, and the second opening 302 of the pool antibacterial device 300 may be arranged in the water cavity 102. During use, when driven by the water pump 201, the water in the water cavity 102 may flow out of the pool water outlet 104. The water may then be conveyed to the pool water inlet 105 through the external piping 202, enter the water flow passage 421 of the pool antibacterial device 300 through the first opening 301, and then sequentially flow through the support frame 420 and the copper substrate 304. The copper substrate 304 may gradually release copper ions under the action of the water flow. The copper ions may be discharged from the second opening 302 with the water flow and flow return to the water cavity 102. The copper ions in the water cavity 102, when reaching a set concentration (greater than 0.3 PPM), can effectively act on the algae or other microorganisms, disrupting the structures and functions of the cell membranes thereof, thereby achieving the killing and inhibition of the microorganisms in the water, and improving the sanitary quality and water cleanliness of the pool.

As shown in FIG. 5, the pool antibacterial device 300 of the above embodiment may include the first housing 400 and the fixing base 410, and the copper substrate 304 may be disposed in the first receiving chamber 401 formed jointly by the first housing 400 and the fixing base 410. Since the second opening 302 may be disposed on the side wall of the first housing 400, the water flow may flow into and out of the first housing 400 through the second opening 302. Accordingly, the pool antibacterial device 300 in this embodiment may not be suitable for installation on the external piping 202 between the pool water outlet 104 and the pool water inlet 105. In view of this, the present disclosure provides another embodiment of the pool antibacterial device 300, where the first opening 301 and the second opening 302 are disposed at opposite ends of a housing 500, respectively, and no other water ports are disposed on the side wall of the housing 500.

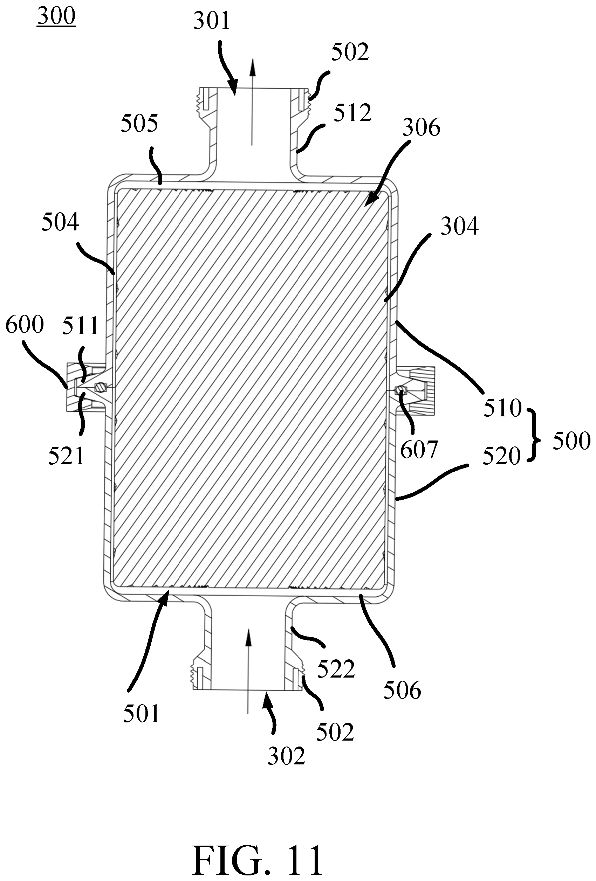

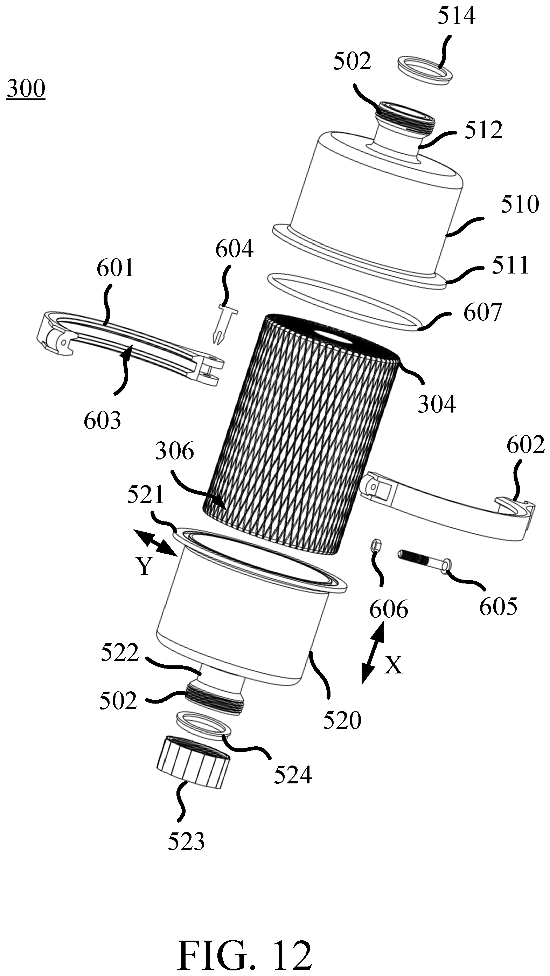

In this embodiment, better shown in FIGS. 11 and 12, both the first opening 301 and the second opening 302 may be connected to the external piping 202, so that the pool antibacterial device 300 may be mounted not only to the pool water outlet 104 (shown in FIG. 8) or to the pool water inlet 105 (shown in FIG. 9), but may also be mounted to the external piping 202 (shown in FIG. 10) between the pool water outlet 104 and the pool water inlet 105.

For example, referring now to FIGS. 11 and 12, the pool antibacterial device 300 may include the housing 500 and a fastening ring 600. The housing 500 may include an upper housing 510 and a lower housing 520. The upper housing 510 may include the first opening 301 and the lower housing 520 may include the second opening 302. The upper housing 510 and the lower housing 520 are each of a hollow structure and are arranged opposite each other in the vertical direction and can be removably connected to each other. The fastening ring 600 can couple together the upper housing 510 to the lower housing 520 to form a complete second receiving chamber 501. The copper substrate 304 may be arranged in the second receiving chamber 501, between the first opening 301 and the second opening 302. At least part of the copper substrate 304 may be in contact with an inner wall of the second receiving chamber 501.

Referring still to FIGS. 11 and 12, the second receiving chamber 501 may be in the form of a hollow cylinder, and the inner wall of the second receiving chamber 501 may include a side wall 504, a top wall 505 and a bottom wall 506. The first opening 301 may be disposed in the top wall 505 and the second opening 302 may be disposed in the bottom wall 506, that is, the first opening 301 and the second opening 302 are arranged symmetrically in the axial direction. The copper substrate 304 may be in the form of a solid cylinder arranged in the second receiving chamber 501 and fitting against at least one of the side wall 504, the top wall 505 and the bottom wall 506 of the second receiving chamber 501, to prevent the water flow in the flow path from the first opening 301 to the second opening 302 from bypassing the copper substrate 304 and flowing through a gap between the copper substrate 304 and the second receiving chamber 501, thereby ensuring that the water flow passes through the mesh structure 306 of the copper substrate 304. Furthermore, in some embodiments, the copper substrate 304 may fit against the side wall 504, the top wall 505, and the bottom wall 506 so as to prevent the water flow from flowing through the gap between the copper substrate 304 and the second receiving chamber 501, which would otherwise affect the antibacterial effect.

In the embodiments described herein, the configuration of the housing 500 with a removable connection between the upper housing 510 and the lower housing 520 may facilitate mounting and dismounting of the copper substrate 304. For example, during mounting, one end of the copper substrate 304 may be mounted into the upper housing 510, the lower housing 520 then may be sleeved over the copper substrate 304 from the other end of the copper substrate 304, and the upper housing 510 and the lower housing 520 then may be connected to each other via the fastening ring 600, thereby enabling a secure mounting of the copper substrate 304 in the second receiving chamber 501.

In this embodiment, the housing 500 may be of a hollow cylindrical structure. However, it should be understood that, in some other embodiments, the housing 500 may have other shapes, such as a truncated cone or any other similar shape without departing from the scope of the present disclosure. The shape of the copper substrate 304 arranged in the second receiving chamber 501 may be the same as or different from the shape of the housing 500, so long as the water flow may flow through the copper substrate 304 when the water flow flows from the first opening 301 to the second opening 302, to achieve the release of the copper ions and the antibacterial treatment of the water.

As shown in FIGS. 11 and 12, the opposite ends of the upper housing 510 and the lower housing 520 may be provided with a first butt-joint portion 511 and a second butt-joint portion 521, respectively. Both the first butt-joint portion 511 and the second butt-joint portion 521 may protrude outwardly in a radial direction Y to form connection portions capable of being butt-jointed to each other.

The fastening ring 600 may include a first arc-shaped portion 601 and a second arc-shaped portion 602. The first arc-shaped portion 601 and the second arc-shaped portion 602 may be assembled to form an annular structure matching the outer diameter of the housing 500 to achieve a fastening and fixing of the housing 500. Inner rings of the first arc-shaped portion 601 and the second arc-shaped portion 602 may each be provided with a fixing groove 603, and in a fastened state, the first butt-joint portion 511 and the second butt-joint portion 521 may be embedded in the fixing grooves 603, thereby achieving an axial locking connection between the upper housing 510 and the lower housing 520. In particular, one end of the first arc-shaped portion 601 and one end of the second arc-shaped portion 602 may be connected to each other via a pin 604, and the other end of the first arc-shaped portion 601 and the other end of the second arc-shaped portion 602 may be removably connected to each other via a bolt 605 and a nut 606, thereby facilitating assembly and disassembly operations.

Furthermore, a sealing ring 607 may be embedded in the fixing grooves 603 of the fastening ring 600. When the upper housing 510 and the lower housing 520 are connected to each other via the fastening ring 600, the second receiving chamber 501 may be sealed by the sealing ring 607 to prevent leakage of the water from the second receiving chamber 501.

Referring still to FIGS. 11 and 12, an end of the upper housing 510 provided with the first opening 301 may extend to form a first protruding pipe 512. The outer diameter of the first protruding pipe 512 may be smaller than the outer diameter of the upper housing 510 for connection with the pool water outlet 104, the pool water inlet 105 or the external piping 202. Accordingly, an end of the lower housing 520 provided with the second opening 302 may extend to form a second protruding pipe 522. The outer diameter of the second protruding pipe 522 may be smaller than the outer diameter of the lower housing 520 for connection with the pool water outlet 104, the pool water inlet 105 or the external piping 202.

As with the previous embodiment, the pool antibacterial device 300 depicted in FIGS. 11 and 12 may be flexibly mounted to either the pool water outlet 104 or the pool water inlet 105, or to each of the pool water outlet 104 and the pool water inlet 105. Furthermore, in some embodiments, the pool antibacterial device 300 may be mounted to the external piping 202 between the pool water outlet 104 and the pool water inlet 105. This embodiment may be explained in three mounting modes: the pool antibacterial device 300 may be mounted only to the pool water outlet 104, only to the pool water inlet 105, or to the external piping 202.

Referring now to FIGS. 8, 11 and 12, in this embodiment, the pool antibacterial device 300 may be mounted to the pool water outlet 104. In particular, the first protruding pipe 512 may be directly butt-joined to and in communication with the pool water outlet 104, and the second protruding pipe 522 may be located in the water cavity 102. During use, the water in the water cavity 102 may be driven by the water pump 201 to enter the pool antibacterial device 300 through the second protruding pipe 522. The water may then sequentially flow through the second opening 302, the copper substrate 304 in the second receiving chamber 501, and the first opening 301, and enter the external piping 202 through the pool water outlet 104. The water flow may pass through the copper substrate 304, since during the flowing process of the water flow, the copper substrate 304 may generate a potential difference on the surface of the copper substrate due to contact with the water, and in turn gradually releases copper ions. The copper ions may sequentially flow into the external piping 202 with the water flow and return to the water cavity 102 through the pool water inlet 105, and the copper ions may gradually accumulate in the water cavity 102 with the circulation flow of the water, until the copper ions in the water cavity 102 reach a set concentration (In some embodiments greater than 0.3 PPM). The copper ions may then bind to the cell membranes of the algae and other microorganisms, disrupting their structure and function, and causing the microorganisms to die or lose their activity, thereby inhibiting the growth of colonies in the pool, and maintaining the cleanliness of the pool.

Furthermore, because the first opening 301 and the second opening 302 of the pool antibacterial device 300 of this embodiment are arranged in a symmetrical configuration, it may be possible to connect in reverse during mounting. For example, the first protruding pipe 512 may be arranged in the water cavity 102, and the second protruding pipe 522 may be connected to the pool water outlet 104. This can also achieve the same water flow path and antibacterial effect, further enhancing the adaptability and flexibility of the pool antibacterial device 300 in on-site implementations.

Referring to FIGS. 9, 11 and 12, in this embodiment, the pool antibacterial device 300 may be mounted to the pool water inlet 105. In particular, the first protruding pipe 512 may be directly butt-joined to and in communication with the pool water inlet 105, and the second protruding pipe 522 may be located in the water cavity 102. During use, when driven by the water pump 201, the water in the water cavity 102 may first enter the external piping 202 through the pool water outlet 104. The water may then enter the first protruding pipe 512 through the pool water inlet 105, flow into the second receiving chamber 501 of the pool antibacterial device 300 through the first opening 301, sequentially flow through the copper substrate 304 between the first opening 301 and the second opening 302 and through the second opening 302, and return to the water cavity 102 from the second protruding pipe 522. The copper substrate 304 generates a potential difference on the surface of the copper substrate during the passage of water flow and gradually releases copper ions. The copper ions may be discharged from the second opening 302 and flows into the water cavity 102 with the water flow. As the water flow circulates, the copper ions in the water cavity 102 may gradually accumulate and when they reach a set concentration value (In some embodiments greater than 0.3 PPM), the copper ions may bind to the cell membranes of the algae and other microorganisms, disrupting their structure and function, thereby causing the death of the microorganisms, inhibiting the growth of colonies in the pool and keeping the water clean.

In some embodiments, because the first opening 301 and the second opening 302 of the pool antibacterial device 300 are symmetrically arranged, the pool antibacterial device 300 may also be mounted in a reverse manner That is, the first protruding pipe 512 may be arranged in the water cavity 102, and the second protruding pipe 522 may be in communication with the pool water inlet 105.

Referring now to FIGS. 10 to 12, in this embodiment, the pool antibacterial device 300 may be mounted to the external piping 202 between the pool water outlet 104 and the pool water inlet 105. It may be possible that the pool antibacterial device 300 may be mounted between the water pump 201 and the pool water outlet 104, or between the water pump 201 and the pool water inlet 105, or that one pool antibacterial device may be mounted between the water pump 201 and the pool water outlet 104, and one pool antibacterial device may be mounted between the water pump 201 and the pool water inlet 105. It should be understood that the embodiment described herein is an example of its installation between the water pump 201 and the pool water inlet 105 and is illustrative in nature, such that other installation configurations may be implemented without departing from the scope of the present disclosure.

Furthermore, in some embodiments, the pool antibacterial device 300 may be mounted to the external piping 202 between the water pump 201 and the pool water inlet 105. That is, the external piping 202 in this case may include a first water pipe 202a, a second water pipe 202b, and a third water pipe 202c in sequential communication with each other. The first water pipe 202a may communicate the pool water outlet 104 with the pump water inlet 201a, the second water pipe 202b may communicate the pump water outlet 201b with the first opening 301 of the pool antibacterial device 300, and the third water pipe 202c may communicate the second opening 302 of the pool antibacterial device 300 with the pool water inlet 105. In other words, the pump water inlet 201a is in fluid communication with the pool water outlet 104 via the first water pipe 202a, the pump water outlet 201b is in fluid communication with the first opening 301 of the pool antibacterial device 300 via the second water pipe 202b, and the second opening 302 of the pool antibacterial device is in fluid communication with the pool water inlet 105 via the third water pipe 202c.

The opposite ends of the second water pipe 202b and the third water pipe 202c may each be provided with an external thread (not shown), and accordingly, the first protruding pipe 512 and the second protruding pipe 522 may also be provided with external threads 502. The first protruding pipe 512 and the second water pipe 202b may be connected by providing a nut 503, and a sealing gasket 514 may be disposed therebetween, so as to achieve a sealed connection between the first protruding pipe 512 and the second water pipe 202b. Likewise, the second protruding pipe 522 and the third water pipe 202c may be threadedly connected to each other via a nut 523, and a sealing gasket 524 which may have the same structure as the sealing gasket 514, may be disposed therebetween, so as to achieve a sealed connection between the second protruding pipe 522 and the third water pipe 202c.

When driven by the water pump 201, the water in the water cavity 102 of the pool 100 may sequentially enter the first water pipe 202a of the external piping 202 from the pool water outlet 104, enter the second water pipe 202b through the pump water inlet 201a and the pump water outlet 201b, then pass through the first protruding pipe 512 and the first opening 301 into the second receiving chamber 501 of the pool antibacterial device 300, and flow through the copper substrate 304 to the second opening 302. The water flow may pass through the copper substrate 304, and the copper substrate 304 may generate a potential difference on the surface of the copper substrate under the action of the water flow and gradually releases copper ions. The copper ions may flow from the second opening 302 into the third water pipe 202c of the external piping 202 and return to the water cavity 102 through the pool water inlet 105 with the water flow. As the water circulates, the copper ions in the water cavity 102 accumulate in the water cavity 102, and when the copper ions reach a set concentration (In some embodiments greater than 0.3 PPM), the copper ions may bind to the cell membranes of the algae and other microorganisms, disrupting their structure and function, thereby causing the death of the microorganisms, inhibiting the growth of colonies in the pool, and achieving the purification of the water in the pool.

Further, the first opening 301 and the second opening 302 of the pool antibacterial device 300 of this embodiment may be symmetrically arranged, so that the pool antibacterial device 300 may also be mounted in a reverse manner at the position of the piping. That is, the second protruding pipe 522 may be connected to the second water pipe 202b, and the first protruding pipe 512 may be connected to the third water pipe 202c, so that the water flow sequentially flows from the second opening 302, passes through the copper substrate 304, and out of the first opening 301, and finally returns to the water cavity 102. In this way, it may be also possible to achieve the function of releasing copper ions and disinfecting water while inhibiting algae growth.

It should be noted that, in addition to the aforementioned mounting mode of the pool antibacterial device 300 having the housing 500, the present disclosure provides another embodiment in which the pool antibacterial device 300 may be adapted to be mounted to the external piping 202. The pool antibacterial device 300 of this embodiment may also be mounted between the water pump 201 and the pool water inlet 105 as shown in FIG. 13. However, unlike the aforementioned structure of the housing, the structural features thereof will be further described in the following embodiment.

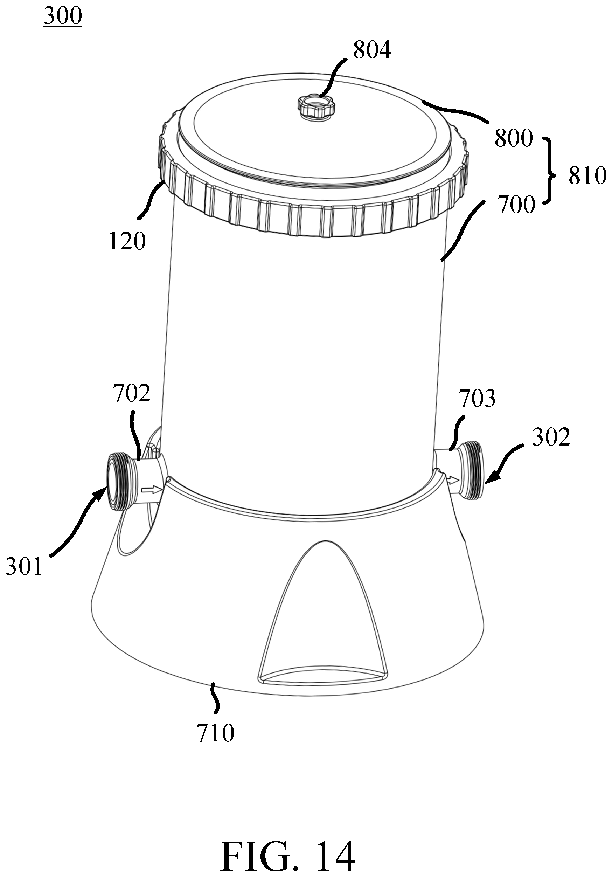

With reference now to FIGS. 14 to 16, in this embodiment, the pool antibacterial device 300 may include a housing 810. The housing 810 may include a second housing 700 and a cover 800. The second housing 700 may be of a cylindrical structure, the interior of which may be hollow, and the second housing 700 and the cover 800 may be sealingly connected to each other to jointly define a third receiving chamber 701. The second housing 700 may be provided with a water intake passage 702 and a water discharge passage 703. One end of the water intake passage 702 may be provided as the first opening 301, which extends outwardly and extends out of the third receiving chamber 701, for connection with the pump water outlet 201b in the external piping 202. Likewise, one end of the water discharge passage 703 may be provided as the second opening 302, which extends outwardly and extends out of the third receiving chamber 701 for connection with the external piping 202 and ultimately for fluid communication with the pool water inlet 105. By way of example, a base 710 may be disposed under the second housing 700. The base 710 may be fixedly connected to the second housing 700, and the base 710 may be configured to securely mount the entire pool antibacterial device 300 to the ground, to ensure that the pool antibacterial device may have a stable structure and may be prevented from shaking during operation.

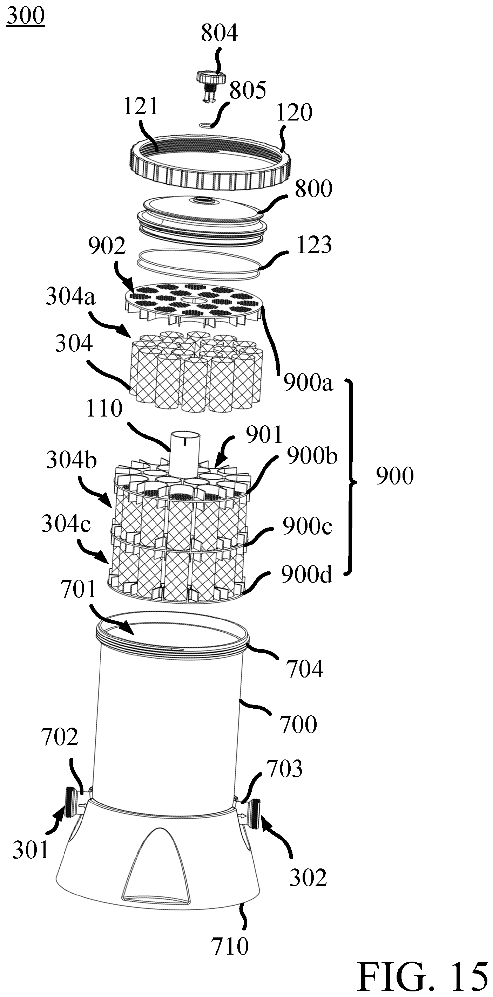

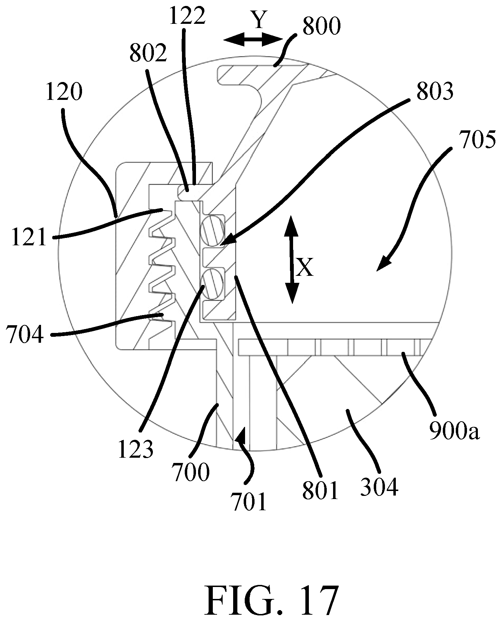

Referring to FIGS. 15 and 17, in this embodiment, the cover 800 may be connected to the second housing 700 by means of a fixing ring 120. The fixing ring 120 may be an annular component with an internal thread 121 on an inner side thereof. An outer side wall of an upper end of the second housing 700 may be provided with an external thread 704 for cooperation with the internal thread 121 to connect the cover 800 to the second housing 700. An edge of the cover 800 may extend downwardly in the vertical direction (i.e., the axial direction X of the second housing 700) to form a cover side wall 801. An upper end of the cover side wall 801 may extend outwardly in the radial direction Y to form an annular flange 802. In the radial direction Y, the fixing ring 120 may be provided with an abutment surface 122 for cooperation with the annular flange 802. When the cover 800 is mounted to the second housing 700, the cover side wall 801 may be first extended into the third receiving chamber 701 until the annular flange 802 comes into contact with an upper end surface of the second housing 700, and then the fixing ring 120 may be screwed in such that the internal thread 121 thereof may be threadedly engaged with the external thread 704 on the outer side wall of the second housing 700. The fixing ring 120 may be moved downwardly in the vertical direction during the tightening process until the abutment surface 122 abuts against the annular flange 802. Such a connection method may provide simple structure and convenient installation.

Further, to improve the sealing performance of the connection between the cover 800 and the second housing 700, the cover side wall 801 may be provided with two annular grooves 803. The annular groove 803 may be configured to receive a sealing ring 123. When the cover 800 and the second housing 700 are fixed by means of the fixing ring 120, the sealing ring 123 may abut against the respective annular groove 803 and an inner side wall of the second housing 700, respectively, to prevent the leakage of the water flow from the connection between the cover 800 and the second housing 700.

It should be noted that the manner in which the cover 800 is connected to the second housing 700 may not be particularly limited by the embodiments of the present disclosure, as long as the cover 800 may be securely connected to the second housing 700, and the leakage of the water flow from the connection between the cover 800 and the second housing 700 can be prevented.

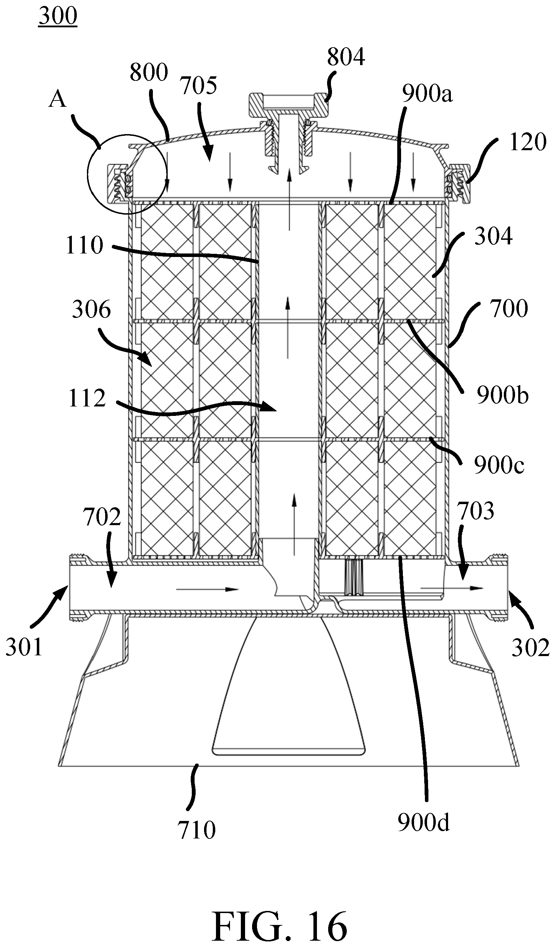

As shown in FIGS. 15 and 16, the cover 800 may be further provided with an exhaust valve 804. During operation of the pool antibacterial device 300, part of air may accumulate in the third receiving chamber 701 and affect the normal flow of the water flow. The user exhausts the air from the third receiving chamber 701 by operating the exhaust valve 804, effectively avoiding the accumulation of air in the third receiving chamber 701, and allowing the water flow to maintain a normal flow path, thereby improving the overall operating efficiency and antibacterial effect of the pool antibacterial device 300. The exhaust valve 804 may be sealingly connected to the cover 800 by a sealing ring 805 to prevent fluid leakage from a mounting part of the exhaust valve 804.

As shown in FIGS. 15 and 16, the pool antibacterial device 300 of this embodiment may further include a plurality of water distribution plates 900 and a communication tube 110 in communication therewith for guiding the flow of the water flow, to improve the efficiency of the release of the copper ions and enhance the antibacterial effect of the water. The plurality of water distribution plates 900 may be located in the third receiving chamber 701 and may be spaced apart from each other in the vertical direction (i.e., in the axial direction X of the second housing 700). In detail, the plurality of water distribution plates 900 are spaced apart from each other in the vertical direction between a first water chamber 705 and a second water chamber 706 both of them will be described below. In this embodiment, the interior of the third receiving chamber 701 may be provided, for example, with four water distribution plates 900, namely, a first water distribution plate 900a, a second water distribution plate 900b, a third water distribution plate 900c, and a fourth water distribution plate 900d in order from top to bottom. Each of the four water distribution plates may be of a circular structure and may fit against an inner wall of the third receiving chamber 701, so as to prevent the water flow from flowing from a gap between the water distribution plate 900 and the inner wall of the third receiving chamber 701.

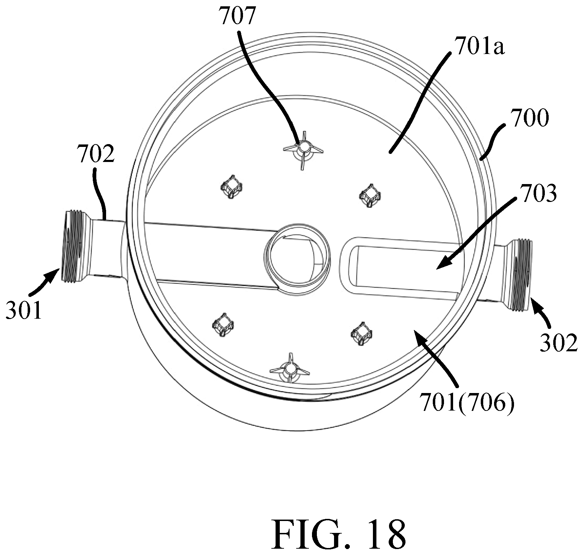

As shown in FIGS. 16 and 18, the cover 800 and the first water distribution plate 900a adjacent thereto may jointly define a first water chamber 705. A bottom wall 701a of the third receiving chamber 701 and the fourth water distribution plate 900d adjacent thereto may jointly define a second water chamber 706. The second water chamber 706 may be in fluid communication with the water discharge passage 703 for discharging the water flow, which flows through the copper substrate, into the water cavity 102 of the pool 100.

As shown in FIGS. 15, 16 and 18, in this embodiment, a plurality of support portions 707 may protrude from the bottom wall 701a of the third receiving chamber 701. The plurality of support portions 707 may support the fourth water distribution plate 900d of the plurality of water distribution plates 900 that is adjacent to the bottom wall 701a, to form the second water chamber 706 between the bottom wall 701a of the third receiving chamber 701 and the fourth water distribution plate 900d, and the support portions 707 may help maintain the stability and layered structure of the water distribution plates 900.

In the embodiments described herein, the water discharge passage 703 may be located in the third receiving chamber 701. In some embodiments, the water discharge passage 703 may be of a groove-like structure, and may be provided in a semi-circular configuration having an upward opening, and the water discharge passage 703 may be in communication with the second water chamber 706 and the third receiving chamber 701, and may be structurally flush with or recessed from the bottom wall 701a of the third receiving chamber 701, so that the water flow entering the second water chamber 706 can flow smoothly into the water discharge passage 703, to ensure the smooth flowing of the water flow.

As shown in FIGS. 15 and 16, the copper substrate 304 may be disposed between any adjacent two water distribution plates 900 of the plurality of water distribution plates 900 for the gradual release of copper ions during the multi-stage passage of the water flow, thereby achieving a continuous antibacterial effect. The communication tube 110 may run through and may be connected to the plurality of water distribution plates 900 to form a connection passage 112. One end of the connection passage 112 may be in fluid communication with the water intake passage 702 and the other end thereof may be in fluid communication with the first water chamber 705, constituting a water flow passage. The copper substrate 304 may be located between the first water chamber 705 and the second water chamber 706 and may be used to fully contact the water as the water flow passes therethrough, to facilitate the release of copper ions. If, for example, there are four water distribution plates, 900, these four water distribution plates 900 may be correspondingly provided with three sets of copper substrates 304. For example, a first set of copper substrates 304a located between the first water distribution plate 900a and the second water distribution plate 900b, a second set of copper substrates 304b located between the second water distribution plate 900b and the third water distribution plate 900c, and a third set of copper substrates 304c located between the third water distribution plate 900c and the fourth water distribution plate 900d. The three sets of copper substrates 304 may be all located between the water intake passage 702 and the water discharge passage 703, so that the water flow entering from the water intake passage 702 sequentially flows through each set of copper substrates 304, releasing copper ions layer by layer to enhance the bactericidal effect.

Further, by way of example, the water intake passage 702 may protrude from the bottom wall 701a of the third receiving chamber 701, may be open at one end opposite to the first opening 301, and may be connected to the communication tube 110 so that the water flow enters the first water chamber 705 only through the communication tube 110, thereby enhancing control over the water flow path. Therefore, the communication tube 110 is connected to the plurality of water distribution plates 900 to guide water between the water intake passage 702, which is in fluid communication with the first opening 301, and the first water chamber 705. The second water chamber 706 is in fluid communication with the second opening 302 via the water discharge passage 703, and in fluid communication with the first water chamber 705 via the mesh structure 306 of the copper substrate 304.

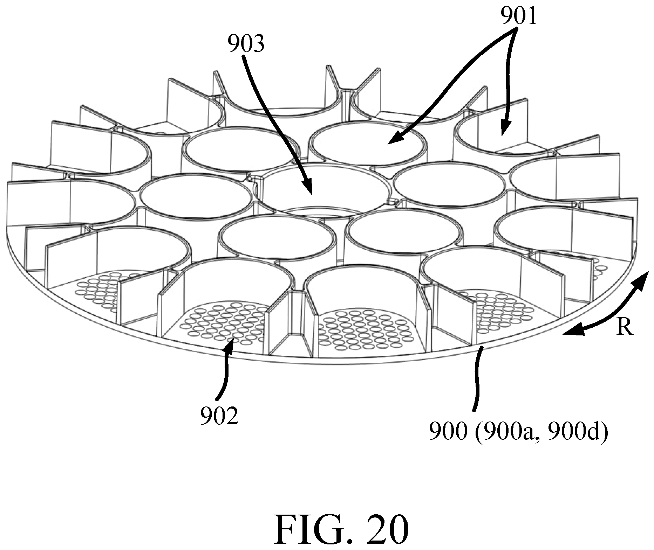

Referring to FIGS. 15, 19 and 20, FIG. 19 shows a perspective view of the second water distribution plate 900b, which may be also the perspective view of the third water distribution plate 900c, and FIG. 20 shows a perspective view of the fourth water distribution plate 900d, which may be also the perspective view of the first water distribution plate 900a after turned 180°. Each of the water distribution plates 900 may be provided with fixing recesses 901 on the surface thereof for insertion of ends of the copper substrates 304 to achieve a secure positioning of the copper substrates 304 within the third receiving chamber 701 and to maintain the desired space arrangement. Further, a bottom wall of each fixing recess 901 may include a plurality of through holes 902 running through the bottom wall of the fixing recess 901, to facilitate fluid communication between the copper substrates 304 adjacent to each other in the vertical direction via the plurality of through holes 902, between the first set of copper substrates 304a and the first water chamber 705, and between the third set of copper substrates 304c and the second water chamber 706. The through holes 902, which may be a perforated structure, may be disposed at the bottoms of the fixing recesses 901 for enhancing fluid communication between the first water chamber 705 and the first set of copper substrates 304a, and between the third set of copper substrates 304c and the second water chamber 706, and also for providing a fluid path between the copper substrates 304 sequentially arranged in the vertical direction, thereby ensuring the continuous and smooth flowing of the water flow in the third receiving chamber 701 during the multi-stage flow of the water, and facilitating the continuous release and diffusion of the copper ions.

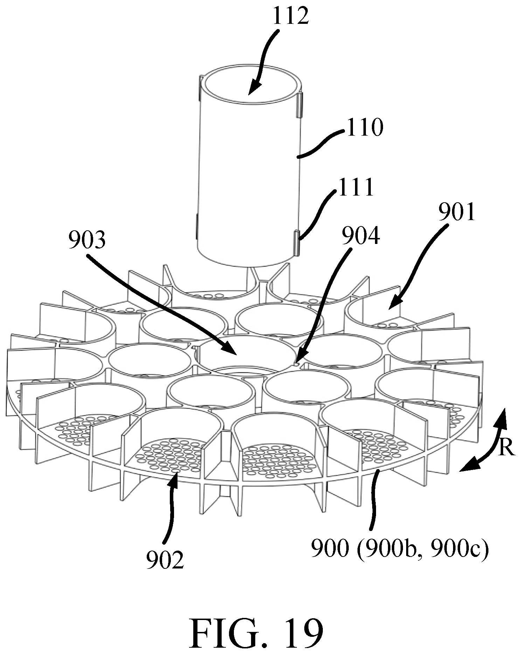

It will be appreciated that, as shown in FIGS. 15 and 20, because the cover 800 may be above the first water distribution plate 900a and no copper substrate 304 may be disposed above the first water distribution plate, the fixing recesses 901 of the first water distribution plate 900a may be only disposed on a lower surface thereof. Likewise, since the bottom wall 701a of the third receiving chamber 701 may be below the fourth water distribution plate 900d, and no copper substrate 304 may be disposed below the fourth water distribution plate, the fixing recesses 901 of the fourth water distribution plate 900d may be only disposed on an upper surface thereof. That may be, the fixing recess 901 may be disposed on one side surface of each of the first water distribution plate 900a and the fourth water distribution plate 900d. As shown in FIGS. 15 and 19, the second water distribution plate 900b and the third water distribution plate 900c may be each located between two adjacent sets of copper substrates 304, and it may be necessary to provide the copper substrates 304 on the upper and lower sides thereof, respectively. Therefore, the upper and lower surfaces of the second water distribution plate 900b and the third water distribution plate 900c may each be provided with fixing recesses 901, to facilitate the mounting and stable positioning of the copper substrates 304.

In this embodiment, preferably four water distribution plates 900 may be provided. However, it should be appreciated that the number of water distribution plates 900 may be set according to the actual structural requirements and the desired copper ion release effect, and may include any number of water distribution plates 900 without departing from the scope of the present disclosure. In other embodiments, the number of water distribution plates 900 may be, for example, set to 2, 3, 5 or more, and accordingly, the number of sets of copper substrates 304 may be adjusted accordingly to one, two, four or more, to adapt to the purification and antibacterial requirements of pools of different sizes.

As shown in FIGS. 15, 19 and 20, a plurality of copper substrates 304 may be disposed between vertically adjacent water distribution plates 900, the plurality of copper substrates 304 constituting one set of copper substrates, to increase the contact area between the water flow and the plurality of copper substrates, thereby enhancing the antibacterial effect. Correspondingly, each of the water distribution plates 900 may be provided with a plurality of fixing recesses 901 for respectively receiving the ends of the plurality of copper substrates 304 to achieve stable positioning thereof. In particular, the center of each water distribution plate 900 may be provided with a mounting portion 903 for connection with the communication tube 110. By way of example, the mounting portion 903 may be of a hollow cylindrical structure, which may run vertically through the water distribution plate 900 and protrude upwardly or downwardly from the surface of the water distribution plate 900. The mounting portion 903 may be vertically flush with the fixing recesses 901 for facilitating structural integration and machining. The fixing recesses 901 are configured to maintain space between adjacent copper substrates of the plurality of copper substrates 304.

As shown in FIG. 19, a plurality of fixing recesses 901 may be disposed at the outer periphery of the mounting portion 903. In some embodiments, six fixing recesses 901 may be uniformly disposed at an inner circle around the mounting portion 903 in the circumferential direction R. Further, twelve fixing recesses 901 may be provided in the circumferential direction R at the outer periphery of the six fixing recesses 901, so that a total of eighteen fixing recesses 901 may be arranged on the side of each water distribution plate 900 for connection with the copper substrates 304. With such an arrangement, eighteen copper substrates 304 may be mounted between each pair of vertically adjacent water distribution plates 900, so that the structure may be compact and the arrangement may be orderly.

The copper substrate 304 in this embodiment may be a solid cylindrical structure, and six fixing recesses 901 arranged at the inner circle surrounding the mounting portion 903 may be correspondingly provided as hollow cylindrical through holes adapted to the solid cylindrical copper substrates 304, whereas twelve fixing recesses 901 arranged at an outer circle may be provided in the shape of a combination of a semicircle and a rectangle, to adapt to the mounting of different copper substrates 304.

In this embodiment, although eighteen fixing recesses 901 may be provided and eighteen copper substrates 304 may be provided between vertically adjacent water distribution plates 900, it should be understood that the specific number of copper substrates 304 and the number of corresponding fixing recesses 901 may include any number of distribution plates 900 without departing from the scope of the present disclosure. For example, in other embodiments, ten, twelve or more copper substrates 304 may be disposed between vertically adjacent water distribution plates 900, depending on the antimicrobial capability requirements, spatial structure, or processing process, and accordingly, a corresponding number of fixing recesses 901 may be provided on one side of each of the water distribution plates 900 to meet the mounting requirements of the copper substrates. In addition, the cross-sectional shape of the copper substrate 304 may not be limited to a solid cylindrical shape, but may be set to a rectangular, semi-circular, elliptical or any other similar shape depending on the use requirements without departing from the scope of the present disclosure. Correspondingly, the structure of the fixing recess 901 can be adjusted accordingly to ensure that the copper substrate 304 can be stably mounted in the fixing recess and to ensure the smooth flowing of the water flow.

As shown in FIG. 19, the mounting portion 903 of each water distribution plate 900 further may include a groove 904, and an outer wall of the communication tube 110 may include a protrusion 111, which may cooperate and engage with the groove 904. That is, the communication tube 110 may be connected to each water distribution plate 900 of the plurality of water distribution plates 900 by means of the cooperation and engagement of the groove 904 with the protrusion 111, so as to ensure the relative fixation of the communication tube 110, and avoid rotation of the communication tube 110 relative to the water distribution plate 900, thereby improving the structural stability. In other embodiments, the mounting portion 903 may include a protrusion (not shown), and the outer wall of the communication tube 110 may include a groove (not shown). The protrusion and the groove may also be engaged with each other to achieve the connection between the communication tube 110 and the water distribution plate 900. It should be further appreciated that, in other embodiments, the communication tube 110 and the water distribution plate 900 may be connected in various other ways without limitation to the specific connection method, such as threaded connection, screw connection and other structural forms.

In the embodiment shown in FIGS. 15 and 16, the communication tube 110 may be formed of three sections of tube, and each section of the communication tube 110 may be connected to the mounting portions 903 of two vertically adjacent water distribution plates 900. However, it should be further understood that, in other embodiments, the communication tube 110 may be a tube of an integral structure, which runs through the plurality of water distribution plates 900, and may have a lower end in communication with the water intake passage 702, and an upper end in communication with the first water chamber 705.

Referring to FIGS. 13, 15 and 16, when the pool antibacterial device 300 is mounted to the external piping 202 between the water pump 201 and the pool water inlet 105, the external piping 202 may include three water pipes connected to each other in sequence, such as a first water pipe 202a, a second water pipe 202b, and a third water pipe 202c. The first water pipe 202a may communicate the pool water outlet 104 with the pump water inlet 201a, the second water pipe 202b may communicate the pump water outlet 201b with the first opening 301 of the pool antibacterial device 300, and the third water pipe 202c may communicate the second opening 302 of the pool antibacterial device 300 with the pool water inlet 105. During operation of the pool antibacterial device 300, water in the water cavity 102 may be drawn by the water pump 201 into the first water pipe 202a through the pool water outlet 104 and may be then conveyed via the second water pipe 202b through the first opening 301 into the water intake passage 702 of the pool antibacterial device 300. Furthermore in these embodiments, the water may enter the connection passage 112 through the water intake passage 702, and flows into the first water chamber 705, the water flow in the first water chamber 705 may flow from the through holes 902 in the first water distribution plate 900a to the corresponding first set of copper substrates 304a, the water may exit the first set of copper substrates 304a and then enter the second set of copper substrates 304b through the through holes 902 of the fixing recesses 901 of the second water distribution plate 900b, the water may then exit the second set of copper substrates 304b and enters the third set of copper substrates 304c through the through holes 902 of the fixing recesses 901 of the third water distribution plate 900c, and finally the water may exit the third set of copper substrates 304c and then flow into the second water chamber 706 through the through holes 902 of the fixing recesses 901 of the fourth water distribution plate 900d, and the water flow may then flow through the water discharge passage 703, the second opening 302 and the third water pipe 202c into the pool water inlet 105 and finally into the water cavity 102. The water flow may sequentially flow through the first set of copper substrates 304a, the second set of copper substrates 304b, and the third set of copper substrates 304c, and may fully contact the copper substrates to release copper ions. When the copper ions in the water cavity 102 reach a set concentration (In some embodiments greater than 0.3 PPM), the copper ions bind to the cell membranes of the algae and other microorganisms, disrupting their structure and function, and causing the inactivation or death of the microorganisms, thereby inhibiting the growth of colonies in the pool, achieving a long-term antibacterial effect of the pool, and keeping the pool clean.

On the basis of the embodiment in which three sets of copper substrates 304 are disposed in the third receiving chamber 701, the present disclosure also provides a further embodiment, the structure of which differs from the above embodiment in that the first set of copper substrates 304a and the second set of copper substrates 304b in the above embodiment may be replaced with a fibrous filter material 130 in this embodiment, thereby increasing the filtration capacity of the water flow while maintaining the water antibacterial function, improving the removal effect of impurities and particles in the water, and enhancing the water purification ability.

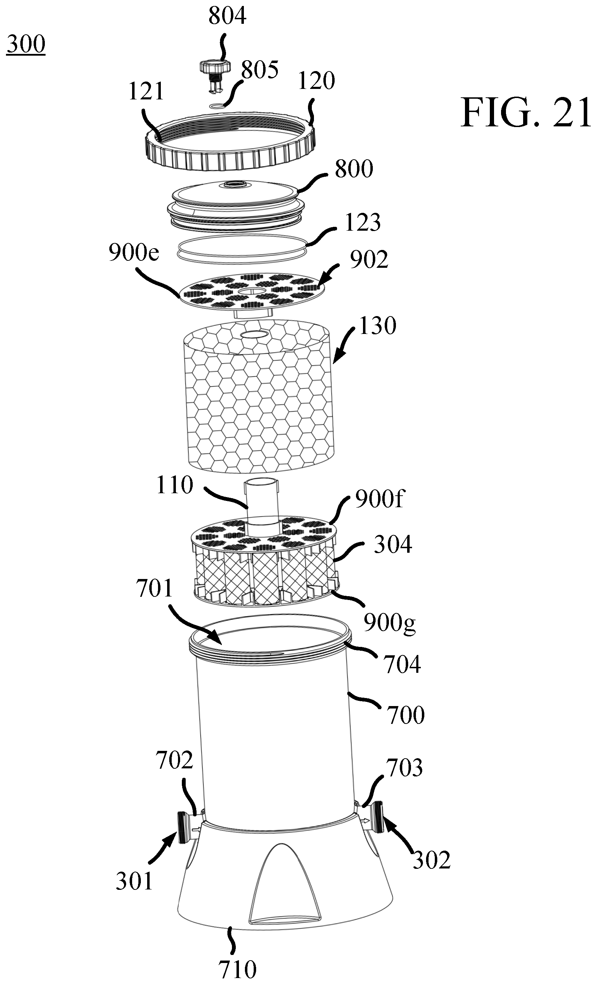

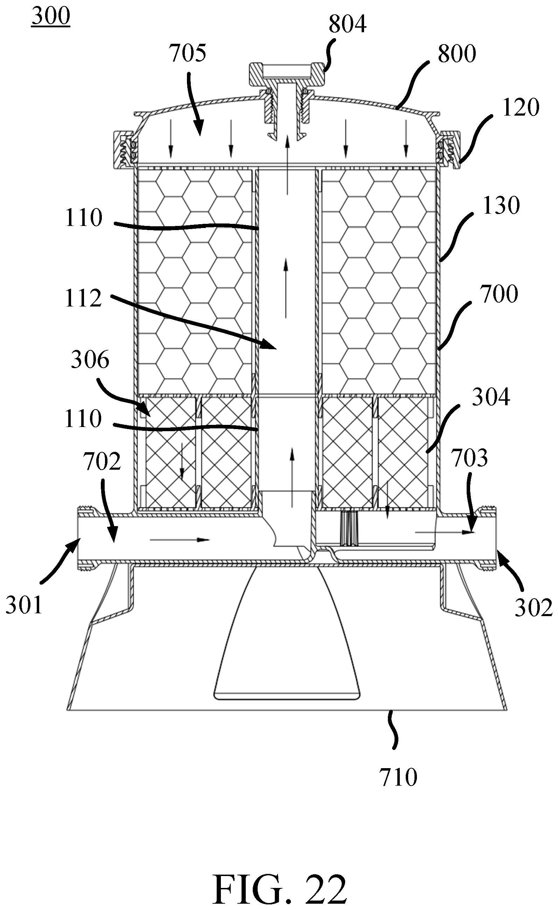

Moreover, referring to FIGS. 18, 21 and 22, the pool antibacterial device 300 may be provided with three water distribution plates 900, namely, a top water distribution plate 900e, an intermediate water distribution plate 900f and a bottom water distribution plate 900g. The top water distribution plate 900e and the cover 800 may jointly define the first water chamber 705, and the bottom water distribution plate 900g and the bottom wall 701a of the third receiving chamber 701 may jointly define the second water chamber 706. A fibrous filter material 130 may be disposed between the top water distribution plate 900e and the intermediate water distribution plate 900f (as shown in figure 21), and a set of copper substrates 304 may be disposed between the intermediate water distribution plate 900f and the bottom water distribution plate 900g. Since the top water distribution plate 900e and the intermediate water distribution plate 900f may be configured to receive the fibrous filter material 130 therebetween, it may be preferable that opposite surfaces of the top water distribution plate 900e and the intermediate water distribution plate 900f may both be planar. Furthermore, it should be appreciated that it may not be necessary to provide any fixing recess 901 for mounting the copper substrate 304, but in order to achieve the continuous passage of the water flow, the top water distribution plate 900e, the intermediate water distribution plate 900f and the bottom water distribution plate 900g may be provided with a plurality of through holes 902, for achieving fluid communication between the first water chamber 705 and the fibrous filter material 130, between the fibrous filter material 130 and the copper substrates 304, and between the copper substrates 304 and the second water chamber 706, respectively.

By way of example, the fibrous filter material 130 may be a plurality of fibrous balls with high specific surface area and filtration efficiency. However, it should be appreciated that, in other embodiments, the fibrous filter material 130 may be in other forms, such as a bundled fibrous filter material, a filamentary fibrous material or other materials with filtration function, the specific type of which can be adjusted according to the filtration requirements, without departing from the scope of the present disclosure.

As shown in FIG. 21, the top water distribution plate 900e and the intermediate water distribution plate 900f may be connected to each other via the communication tube 110, and the fibrous filter material 130 may be filled in a chamber between the top water distribution plate 900e and the intermediate water distribution plate 900f, and may be arranged around the communication tube 110, thereby achieving a good filtration effect.

In this embodiment, two sections of communication tube 110 may be provided, which may be connected to each other. One section of communication tube 110 connects the top water distribution plate 900e to the intermediate water distribution plate 900f, and may have one end in fluid communication with the first water chamber 705. The other section of communication tube 110 connects the intermediate water distribution plate 900f to the bottom water distribution plate 900g, and may be in fluid communication with the water intake passage 702. By means of the two sections of communication tube 110, the first water chamber 705 may be in fluid communication with the water intake passage 702. Moreover, the through holes 902 in the top water distribution plate 900e, the intermediate water distribution plate 900f and the bottom water distribution plate 900g allow the water flow to flow from the first water chamber 705 into the second water chamber 706. The following process may thus be achieved: the water flow passes from the first water chamber 705 through the fibrous filter material 130 for filtration treatment, then through the copper substrates 304, and finally into the second water chamber 706, completing the composite treatment process of filtration and antibacterial. During contact of the copper substrates with the water, copper ions may be released.

It should be noted that a combination structure of one set of copper substrates 304 and a fibrous filter material 130 may be provided to achieve the function mode of filtration followed by antibacterial. However, it should be further understood that, in some embodiments, a combination structure of a plurality of sets of copper substrates 304, such as two, three or more sets, and a fibrous filter material 130 may also be provided according to usage requirements. Likewise, the fibrous filter material 130 may be arranged between a plurality of adjacent water distribution plates 900 to further enhance the filtration effect or prolong the service life.

Referring to FIGS. 13, 21 and 22, when the pool antibacterial device 300 of this embodiment is mounted to the external piping 202 between the water pump 201 and the pool water inlet 105, the external piping 202 sequentially may include a first water pipe 202a, a second water pipe 202b and a third water pipe 202c. The first water pipe 202a communicates the pool water outlet 104 with the pump water inlet 201a. The second water pipe 202b may communicate the pump water outlet 201b with the first opening 301 of the pool antibacterial device 300. The third water pipe 202c may communicate the second opening 302 of the pool antibacterial device 300 with the pool water inlet 105. During operation of the pool antibacterial device 300 of this embodiment, water in the water cavity 102 may be drawn by the water pump 201 into the first water pipe 202a through the pool water outlet 104, and may be conveyed from the first opening 301 to the water intake passage 702 of the pool antibacterial device 300 through the second water pipe 202b, and the water in the water intake passage 702 may enter the first water chamber 705 through the connection passage 112. The water flow may flow from the first water chamber 705 through the through holes 902 in the top water distribution plate 900e into the fibrous filter material 130 under the top water distribution plate to achieve the preliminary water filtration treatment. After filtered by the fibrous filter material 130, the water flow may pass through the through holes 902 of the fixing recesses 901 of the intermediate water distribution plate 900f into the region of the copper substrates 304 disposed under the intermediate water distribution plate, and copper ions may be released as the water flows through the copper substrates 304, thereby achieving effective inhibition of microorganisms in the water.

Subsequently, the water flow may enter the second water chamber 706 through the through holes 902 of the fixing recesses 901 of the bottom water distribution plate 900g, enter the third water pipe 202c through the water discharge passage 703 and the second opening 302, and finally re-enter the water cavity 102 through the pool water inlet 105. When the water flow sequentially passes through the fibrous filter material 130 and the copper substrates 304, the composite effect of "filtration followed by antibacterial" can be achieved. The concentration of copper ions in the water may gradually increase, and may reach a set concentration threshold (e.g., greater than 0.3 PPM) in the water cavity 102. The copper ions at this concentration can bind to the cell membranes of the algae and other microorganisms, disrupting their structure and function, and causing the inactivation or death of the microorganisms, thereby effectively inhibiting the growth of colonies in the pool, and achieving lasting cleaning and antibacterial effects of the water.

In summary, in the above embodiments of the pool antibacterial device 300 according to the present disclosure, by providing the copper substrate 304, copper ions may be released continuously during circulation of the water, achieving a long-lasting and stable antibacterial function. It will be appreciated that the copper substrate 304 may have a wide range of antibacterial effects and can effectively inhibit the growth of microorganisms such as bacteria. This antibacterial property does not decay over time, may have long-term use capability, and significantly reduces maintenance frequency. The copper substrate 304 maintains a good antibacterial effect even if oxidation or attachment of impurities occurs on the surface. The copper substrate 304 may be friendly to the human body and the environment due to the low toxicity and environmentally friendly properties. Compared with many chemical disinfectants and antibacterial agents, the use of the copper substrate 304 avoids the persistent pollution and drug resistance problems that may be caused by traditional chemical disinfectants. Therefore, the pool antibacterial device 300 according to the embodiments of the present disclosure may not only be reasonably structured and easy to mount, but can also effectively reduce maintenance costs while ensuring water cleanliness, and may be suitable for frame pools, inflatable massage pools, home swimming pools, landscape pools, and many other implementations.

The antibacterial principles of the copper substrate 304 may be as follows.
1. Copper ions can bind to cell membranes of microorganisms, disrupting their structure and function, causing the cell membranes to become more permeable, triggering the leakage of cell contents, and ultimately causing the death of the microorganisms.
2. The copper ions can bind to various enzyme molecules inside bacteria, inhibit their normal catalytic activity, interfere with the metabolism process of the bacteria, and thus inhibit the growth and reproduction of the bacteria.
3. The copper ions can promote the generation of reactive oxygen species (ROS) such as superoxide anions and hydrogen peroxides. The reactive oxygen species can oxidatively damage cell membranes, proteins, DNA and other biological macromolecules of the bacteria, thereby causing apoptosis or lysis of the bacteria.
4. The copper ions can directly embed into the cell membranes of the bacteria, to change the integrity and fluidity of the membranes, causing the cells to lose their barrier function and leak important intracellular components, thereby causing the bacteria to lose their ability to survive.
5. The antibacterial properties of the copper substrate can also inhibit the formation of biofilms and the ability of bacteria to aggregate. The biofilms, as protective structures for bacteria, generally have strong drug resistance and persistence. The action of the copper ions can effectively disrupt their formation process and reduce the attachment and growth of microorganisms from the source.

The copper substrate 304 described above may be made of various materials, such as red copper (content > 99.5%), brass or copper alloy. In terms of structural morphology, in addition to the cylindrical shape in the four embodiments described above, the copper substrate 304 may be of a mesh structure, a spherical structure, a sheet-like structure or other geometries that can increase the contact area and facilitate the passage of the water flow.

Regardless of the material or structure of the copper substrate 304, its dosage criteria need to meet the following conditions to ensure a good antibacterial effect: no less than 150 g of copper substrate per 1000 L of water, or a contact area of no less than 0.3 m² of copper substrate per 1000 L of water. In addition, in order to promote the full release of copper ions, the velocity of the water flow flowing through the copper substrate may be required to be no less than 0.8 m/s.

The antibacterial effect of the copper substrate 304 may be as follows.
1. With a water capacity of 669 L in the water cavity 102 of the pool 100, when the concentration of the copper ions in the water reaches 0.61 PPM, the total number of colonies in the water decreases from 2840 cfu/ml to 40 cfu/ml after 6 consecutive days of use of the copper substrate 304 at that concentration.
2. With a water capacity of 1440 L in the water cavity 102 of the pool 100, when the concentration of the copper ions in the water reaches 0.78 PPM, the total number of colonies in the water decreases from 20600 cfu/ml to 10 cfu/ml after 6 consecutive days of use of the copper substrate 304 at that concentration.

The present disclosure describes the structure of the pool antibacterial device 300 and the effect of the copper substrate 304 in the antibacterial treatment of the water. By providing the copper substrate 304, the pool antibacterial device 300 can reduce the safety hazards during use of the user, the frequency of use of chemical disinfectants, and system maintenance costs, while ensuring water cleanliness and microbiological control, thereby achieving the purpose of energy-saving, efficient and environmentally friendly water antibacterial treatment, and having good practicality and promotion value.

Although the present disclosure has been illustrated and described with reference to some preferred embodiments of the present disclosure, those of ordinary skill in the art should understand that the above contents are further detailed descriptions for the present disclosure with reference to specific embodiments.

The foregoing descriptions are only embodiments of the present disclosure and are not intended to limit the present disclosure. For those skilled in the art, various changes and variations can be made to the present disclosure. Any modifications, equivalent replacements, and improvements made without departing from the principle of the present disclosure shall fall within the scope of the claims of the present disclosure.

Furthermore, it should be apparent that the present disclosure is not limited to the details of the above-mentioned exemplary embodiments, and the present disclosure can be implemented in other specific forms without departing from the basic features of the present disclosure. Therefore, no matter from which point of view, the embodiments should all be regarded as exemplary and non-limiting. The scope of the present disclosure is defined by the appended claims rather than the above-mentioned description, and therefore it is intended that all changes which fall within the meaning and range of equivalents of the claims are embraced in the present disclosure. Any reference signs in the claims should not be construed as limiting the claims involved. In addition, it is apparent that the word "comprise/include" does not exclude other elements or steps, and the singular does not exclude the plural. The terms first, second, etc. are used for designations and do not represent any particular order.

It should be understood that the embodiments as shown in the drawings only show the optional shapes, sizes and arrangements of optional components of the inflatable bed and inflatable products according to the present disclosure, which are merely illustrative but not restrictive, and other shapes, sizes and arrangements may be employed without departing from the idea and scope of the present disclosure.

## Claims

1. A pool antibacterial device comprising:
a housing (430, 500, 810) comprising a first opening (301) and a second opening (302); and
at least a copper substrate (304) disposed within the housing (430, 500, 810), between the first opening (301) and the second opening (302), and comprising a mesh structure (306) configured to allow water to pass therethrough for an antibacterial treatment of water; the first opening (301) and the second opening (302) being in fluid communication with each other through the mesh structure (306) of the copper substrate (304).

2. The pool antibacterial device of claim 1, wherein the housing (430) further comprises:
a fixing base (410) comprising the first opening (301); and
a first housing (400) connected to the fixing base (410) to define a first receiving chamber (401), the first housing (400) further comprising the second opening (302); and
the pool antibacterial device (300) further comprises:
a support frame (420) disposed within the first receiving chamber (401), connected to the fixing base (410) and in fluid communication with the first opening (301), wherein the support frame (420) has a grid structure (420a); and the copper substrate (304) is placed inside the first housing (400) and disposed around the support frame (420); the mesh structure (306) of the copper substrate (304) being in fluid communication with the grid structure (420a) and the second opening (302).

3. The pool antibacterial device of claim 1, wherein the housing (500) comprises:
an upper housing (510) comprising the first opening (301); and
a lower housing (520) connected to the upper housing (510) to form a second receiving chamber (501), the lower housing (520) further comprising the second opening (302); and
the copper substrate (304) is disposed within the second receiving chamber (501) between the first opening (301) and the second opening (302), and at least part of the copper substrate (304) is in contact with an inner wall of the second receiving chamber (501).

4. The pool antibacterial device of claim 1, wherein the housing (810) further comprises:
a second housing (700) comprising the first opening (301) and the second opening (302); and
a cover (800) sealingly connected to the second housing (700) to define a third receiving chamber (701); and
the pool antibacterial device (300) further comprises:
a plurality of water distribution plates (900) spaced apart from each other in a vertical direction between a first water chamber (705) and a second water chamber (706) and disposed within the third receiving chamber (701), the copper substrate (304) being disposed between adjacent water distribution plates (900a, 900b, 900c, 900d, 900e, 900f, 900g) of the plurality of water distribution plates (900).

5. The pool antibacterial device of claim 4, wherein a communication tube (110) is connected to the plurality of water distribution plates (900) to guide water between a water intake passage (702), in fluid communication with the first opening (301), and the first water chamber (705),
wherein the second water chamber (706) is in fluid communication with the second opening (302) via a water discharge passage (703), and in fluid communication with the first water chamber (705) via the mesh structure (306) of the copper substrate (304).

6. The pool antibacterial device of claim 4, wherein at least one water distribution plate (900a) of the plurality of water distribution plates (900), positioned adjacent to the cover (800), and the cover (800) jointly define the first water chamber (705); and
at least one other water distribution plate (900d) of the plurality of water distribution plates (900), positioned adjacent to a bottom wall (701a) of the third receiving chamber (701), and the bottom wall (701a) of the third receiving chamber (701) jointly define the second water chamber (706).

7. The pool antibacterial device of claim 5, further comprising a fibrous filter material (130) disposed between at least a pair of adjacent water distribution plates (900e, 900f) of the plurality of water distribution plates (900) and around the communication tube (110),
wherein the first water chamber (705) is in fluid communication with the second water chamber (706) via the mesh structure (306) of the copper substrate (304) and the fibrous filter material (130).

8. The pool antibacterial device of claim 6, wherein a support portion (707) protrudes from the bottom wall (701a) of the third receiving chamber (701), and the support portion (707) supports the at least one other water distribution plate of the plurality of water distribution plates (900) positioned adjacent to the bottom wall.

9. The pool antibacterial device of claim 4, wherein each of the plurality of water distribution plates (900) comprises a mounting portion (903), the mounting portion comprising one of a groove (904) and a protrusion (111); and an outer wall of the communication tube (110) comprises the other of the groove (904) and the protrusion (111), and the communication tube (110) is connected to each of the plurality of water distribution plates (900) through the cooperation of the groove (907) and the protrusion (111).

10. The pool antibacterial device of claim 7, wherein each of the plurality of water distribution plates (900) comprises at least a fixing recess (901) in which the copper substrate (304) is arranged; said fixing recess (901) is configured to maintain space between adjacent copper substrates of the plurality of copper substrates (304).

11. The pool antibacterial device of claim 10, wherein a bottom wall of the fixing recess (901) comprises a plurality of through holes (902), the copper substrates (304) adjacent to each other in the vertical direction are in fluid communication via the plurality of through holes (902), and the fibrous filter material (130) adjacent to the copper substrate (304) in the vertical direction is in fluid communication with the copper substrate (304) via the plurality of through holes (902).

12. A pool comprising:
a pool body (101) comprising a side wall (103) and a bottom wall (106), the side wall (103) and the bottom wall (106) jointly defining a water cavity (102), and the side wall (103) comprising a pool water inlet (105) and a pool water outlet (104);
an external piping (202);
a water pump (201) disposed outside the water cavity (102) along the external piping (202) and comprising a pump water inlet (201a) and a pump water outlet (201b), the pump water inlet (201a) being in fluid communication with the pool water outlet (104), and the pump water outlet (201b) being in fluid communication with the pool water inlet (105); and
a pool antibacterial device (300) according to one or more of claims 1 to 8, mounted to at least one of the pool water inlet (105), the pool water outlet (104) and the external piping (202) and comprising:
a housing (430, 500, 810) comprising a first opening (301) and a second opening (302); and
a copper substrate (304) disposed within the housing (430, 500, 810) and comprising a mesh structure (306) configured to allow water to pass therethrough, the first opening (301) and the second opening (302) being in fluid communication with each other through the mesh structure (306).

13. The pool of claim 12, wherein the at least one of the pool water inlet (105) and the pool water outlet (104) is in communication with the first opening (301), and the second opening (302) is located in the water cavity (102).

14. The pool of claim 12 or 13, wherein the pool antibacterial device (300) is mounted at the pool water outlet (104) such that water exiting the water cavity (102) passes through the copper substrate (304) prior to entering the water pump (201).

15. The pool of claim 12 or 13, wherein the pool antibacterial device (300) is mounted at the pool water inlet (105) such that water returning to the water cavity (102) passes through the copper substrate (304) before entering the water cavity (102).

16. The pool of claim 12, wherein the pool antibacterial device (300) is mounted on the external piping (202) between the water pump (201) and the pool water inlet (105) or between the water pump (201) and the pool water outlet (104).
